# EUROPEAN PATENT APPLICATION

(11) **EP 2 907 874 A1**
(43) Date of publication of application: **19.08.2015**
(21) Application number: 14155184.6
(22) Date of filing: 14.02.2014
(51) Int. Cl.: C12N 15/113, A61K 31/712, C12Q 1/68, C07H 21/04, A61P 17/00, A61P 17/06

(54) **MiR-21-3p inhibitors in skin disorders**

(71) Applicant: Université de Lausanne, 1011 Lausanne (CH)
(72) Inventor: Degueurce, Gwendoline, 1007 Lausanne (CH); D'Errico, Ilenia, 1004 Lausanne (CH); Michalik, Liliane, 1030 Bussigny (CH); Wahli, Walter, 1112 Echichens (CH); Montagner, Alexandra, 31500 Toulouse (FR)
(74) Representative: reuteler & cie SA

(57) **Abstract**

The present invention is related to miR-21-3p inhibitors, which are particularly useful in the prevention and/or treatment of skin disorders.

SEQUENCE LISTING

<110>  Université de Lausanne

<120>  MiR-21-3p INHIBITORS IN SKIN DISORDERS

<130>  P1769EP00

<160>  28

<170>  PatentIn version 3.5

<210>  1
<211>  21
<212>  RNA
<213>  Homo sapiens

<400>  1
caacaccagu cgaugggcug u                                                21


<210>  2
<211>  22
<212>  RNA
<213>  Mus musculus

<400>  2
caacagcagu cgaugggcug uc                                               22


<210>  3
<211>  21
<212>  DNA
<213>  Artificial Sequence

<220>
<223>  complementary sequence of human miR-21-3p

<400>  3
acagcccatc gactggtgtt g                                                21


<210>  4
<211>  15
<212>  DNA
<213>  Artificial Sequence

<220>
<223>  Human miR-21-3p inhibitor 1

<400>  4
ccatcgactg gtgtt                                                       15


<210>  5
<211>  18
<212>  DNA
<213>  Artificial Sequence

<220>
<223>  Human miR-21-3p inhibitor 2

<400>  5
agcccatcga ctggtgtt                                                    18

<210>  6
<211>  21
<212>  DNA
<213>  Artificial Sequence

<220>
<223>  complementary sequence of murine miR-21-3p

<400>  6
acagcccatc gactgctgtt g                                                21


<210>  7
<211>  15
<212>  DNA
<213>  Artificial Sequence

<220>
<223>  Murine miR-21-3p inhibitor 1

<400>  7
ccatcgactg ctgtt                                                       15


<210>  8
<211>  18
<212>  DNA
<213>  Artificial Sequence

<220>
<223>  Murine miR-21-3p inhibitor 2

<400>  8
agcccatcga ctgctgtt                                                    18


<210>  9
<211>  15
<212>  DNA
<213>  Artificial Sequence

<220>
<223>  basic sequence of mmu-miR-21a-3p_3MM without FAM labelling

<400>  9
ccctagactg ctctt                                                       15


<210>  10
<211>  21
<212>  DNA
<213>  Artificial Sequence

<220>
<223>  mmu-Gapdh mRNA forward primer

<400>  10
gtatgactcc actacggcaa a                                                21


<210>  11
<211>  17
<212>  DNA
<213>  Artificial Sequence

<220>
<223>  mmu-Gapdh mRNA reverse primer

<400>  11
ttcccattct cggcttg                                                      17


<210>  12
<211>  18
<212>  DNA
<213>  Artificial Sequence

<220>
<223>  mmu-Ef1a1 mRNA forward primer

<400>  12
cctggcaagc ccatgtgt                                                     18


<210>  13
<211>  21
<212>  DNA
<213>  Artificial Sequence

<220>
<223>  mmu-Ef1a1 mRNA reverse primer

<400>  13
tcatgtcacg aacagcaaag c                                                 21


<210>  14
<211>  17
<212>  DNA
<213>  Artificial Sequence

<220>
<223>  mmu-Ppar beta/delta mRNA forward primer

<400>  14
cggcagcctc aacatgg                                                      17


<210>  15
<211>  23
<212>  DNA
<213>  Artificial Sequence

<220>
<223>  mmu-Ppar beta/delta mRNA reverse primer

<400>  15
agatccgatc gcacttctca tac                                               23


<210>  16
<211>  23
<212>  DNA
<213>  Artificial Sequence

<220>
<223>  hsa-Ppar beta/delta mRNA forward primer

<400>  16

gcatgaagct ggagtacgag aag                                              23

<210>  17
<211>  20
<212>  DNA
<213>  Artificial Sequence

<220>
<223>  hsa-Ppar beta/delta mRNA reverse primer

<400>  17
gcatccgacc aaaacggata                                                   20

<210>  18
<211>  17
<212>  DNA
<213>  Artificial Sequence

<220>
<223>  mmu-Ppar beta/delta mRNA forward primer

<400>  18
cggcagcctc aacatgg                                                      17

<210>  19
<211>  23
<212>  DNA
<213>  Artificial Sequence

<220>
<223>  mmu-Ppar beta/delta mRNA reverse primer

<400>  19
agatccgatc gcacttctca tac                                              23

<210>  20
<211>  20
<212>  DNA
<213>  Artificial Sequence

<220>
<223>  hsa-P21 mRNA forward primer

<400>  20
ctgtcactgt cttgtaccct                                                   20

<210>  21
<211>  21
<212>  DNA
<213>  Artificial Sequence

<220>
<223>  hsa-P21 mRNA reverse primer

<400>  21
ggtagaaatc tgtcatgctg g                                                 21

<210>  22
<211>  21

<212>  DNA
<213>  Artificial Sequence

<220>
<223>  hsa-P15 mRNA forward primer

<400>  22
cgtgggaaag aagggaagag t                                                21


<210>  23
<211>  15
<212>  DNA
<213>  Artificial Sequence

<220>
<223>  hsa-P15 mRNA reverse primer

<400>  23
ccccagacgc gcagc                                                       15


<210>  24
<211>  20
<212>  DNA
<213>  Artificial Sequence

<220>
<223>  hsa-pri-miR-21 forward primer

<400>  24
ttttgttttg cttgggagga                                                  20


<210>  25
<211>  20
<212>  DNA
<213>  Artificial Sequence

<220>
<223>  hsa-pri-miR-21 reverse primer

<400>  25
agcagacagt caggcaggat                                                  20


<210>  26
<211>  27
<212>  DNA
<213>  Artificial Sequence

<220>
<223>  basic sequence of miR-21-3p LNA probe without Dig labelling

<400>  26
gacagcccat cgactgcact gctgttg                                          27


<210>  27
<211>  21
<212>  DNA
<213>  Artificial Sequence

<220>
<223>  basic sequence of Scramble-miR LNA probe without Dig labelling

<400>  27
gtgtaacacg tctatacgcc a                                                 21

<210>  28
<211>  24
<212>  RNA
<213>  Homo sapiens

<400>  28
ucacaaccuc cuagaaagag uaga                                              24

## Description

### Field of the Invention

The present invention relates to miR-21-3p inhibitors useful for treatment of skin disorders.

### Background of the Invention

Psoriasis is a chronic inflammatory skin disease affecting 1 to 3 % of the Caucasian population with a considerable negative impact on the patient's quality of life. This disease causes red scaly patches on the skin that are areas of inflammation. Over time, affected skin can become resistant to treatment, especially when topical corticosteroids are used.

MicroRNAs (miRNAs) are endogenous small RNAs which can target messenger RNAs (mRNAs) and generate their degradation or prevent their translation. Before complete maturation of miRNA, the endoribonuclease DICER1 generates a double-stranded RNA fragment constituted of miRNA-5p and miRNA-3p strands (the guide strand and the passenger strand). The guide strand is selected by an RNase Argonaute on the basis of the thermodynamic stability of the 5'-end. While the regulatory functions of the guide strands loaded into the RISC complex have been largely described, passenger strands were thought to be degraded and their functions have been largely discounted. MiRNAs are involved in several human pathologies (Bartel, 2004, Cell, 116(2):281-97 *;* Alvarez-Garcia et al, Development, 132(21): 4653-62*)* as well as in skin development, homeostasis and disease (Schneider, 2011, Br J Dermatol, 166(1):22-8)*.*

Up till now few studies have analyzed the expression of microRNAs in the skin. One study showed that the microRNA "miR-203" is highly expressed in the skin and overexpressed in psoriatic skin (Sonkoly et al, 2007, PLoS One, 2(7): e610)*.* Meisgen et al (Exp Dermatol, 2012, 21(4):312-4) showed that miR-21-5p (commonly named miR-21) is upregulated in psoriasis and suppresses T cell apoptosis. Syed et al (2013, Curr Drug Targets, 14(10): 1128-34) demonstrated involvement of a few microRNAs, including miR-21-5p in skin response to UV radiation.

Recently, expression of miR-21-3p was demonstrated to be higher in tissues and serum among non-small cell lung carcinoma patients compared to healthy volunteers (Jiang et al, 2013, Mol Cell Biochem, 383: 67-75*).* Aure et al (2013, Genome Biology, 14:R126) studied individual and combined effects of DNA methylation and copy number alterations on miRNA expression in breast tumors. Their results suggest that miR-21-3p may play an oncogenic role in breast cancer. Lo et al (PLoS One, 2013, 8(9):e75628) showed that miR-21-3p inhibits hepatoma cell growth and functions as a tumor suppressor in hepatocellular carcinoma.

Despite growing interest and efforts in developing therapeutics from miRNAs, there remains a need for new therapeutic treatments for skin disorders including, in particular, inflammatory skin disorders, UV damages, skin cancers.

### Summary of the Invention

The present invention is mainly directed towards miR-21-3p inhibitors and the use thereof, in particular in the prevention and/or treatment of skin disorders.

A first aspect of the invention provides miR-21-3p inhibitors.

A second aspect of the invention relates to compositions comprising miR-21-3p inhibitors.

A third aspect of the invention relates to miR-21-3p inhibitors for use as a medicament. A fourth aspect concerns the use of inhibitors of miR-21-3p for the preparation of a medicament.

A fifth aspect concerns a pharmaceutical preparation comprising miR-21-3p inhibitors or a vector comprising a nucleic acid encoding said miR-21-3p inhibitors, and a pharmaceutically acceptable carrier.

A sixth aspect relates to miR-21-3p inhibitors for use in the prevention and/or treatment of skin disorders.

A seventh aspect provides the use of said miR-21-3p inhibitors for the manufacture of a medicament for the prevention and/or treatment of skin disorders.

An eighth aspect of the invention relates to a method of preventing and/or treating skin disorders, said method comprising administering in a subject in need thereof a miR-21-3p inhibitor, or a pharmaceutical formulation thereof.

A ninth aspect of the invention relates to an *ex-vivo* method of prognosis and/or diagnosis of a skin disorder in a subject comprising determining, in a biological sample of said subject, the level of miR-21-3p.

Other features and advantages of the invention will be apparent from the following detailed description.

### Description of the figures

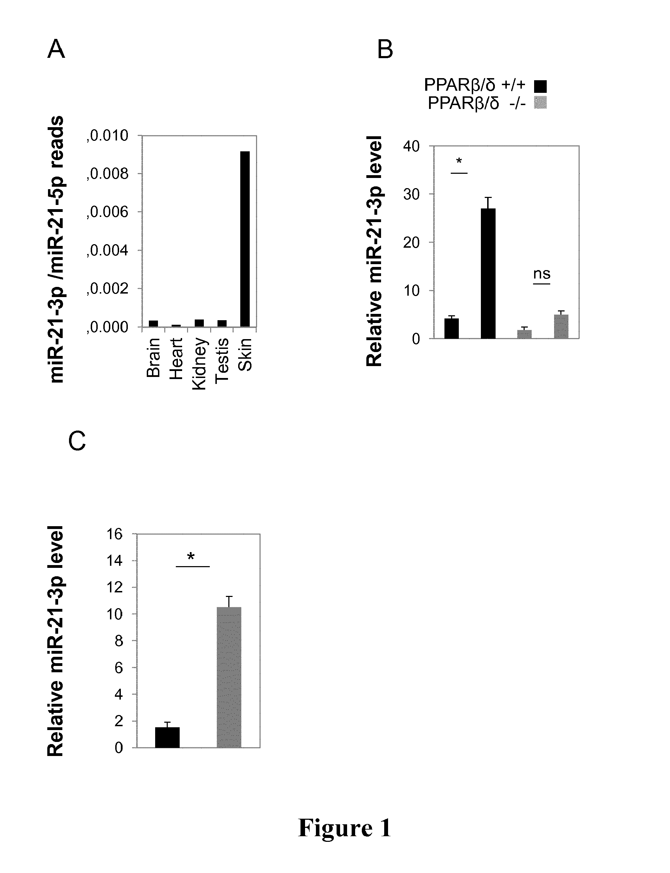

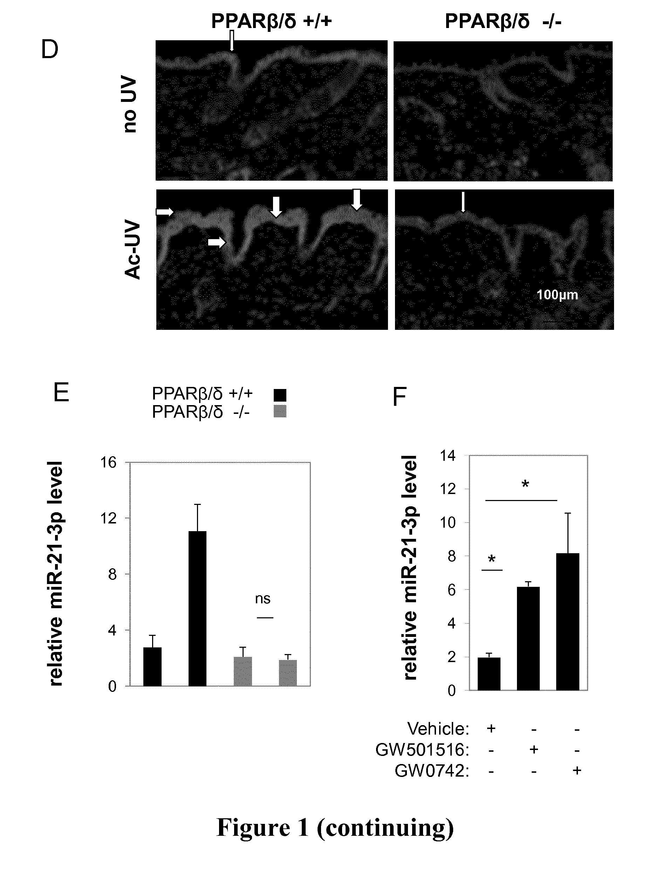

**Figure 1** **shows that PPARβ/δ regulates the expression of UV-induced epidermal miR-21-3p, a miRNA also up regulated in human skin carcinomas.** (A) miR-21-3p/miR-21-5p ratio: number of miR-21-3p reads on the number of miR-21-5p reads obtained from RNA sequencing in mouse total skin, kidney, testis, brain and heart. (B) RT-qPCR quantification of relative miR-21-3p levels in total skin of chronically irradiated (Chr-UV) and non-irradiated PPARβ/δ +/+ (black) and PPARβ/δ -/- (grey) mice. (*) pvalue<0.01, (ns) for non-significant, N=4. (C) RT-qPCR quantification of relative miR-21-3p levels in human normal skin (black) and squamous cell carcinomas (SCC) (grey). (*) pvalue<0.01, N=3. (D) miR-21-3p *in situ* hybridization (light signal, also indicated by the arrows) in the skin of acutely irradiated (Ac-UV) (bottom line) and non-irradiated (no UV) (upper line) PPARβ/δ +/+ (left hand column) and PPARβ/δ -/(right hand column) mice. (E) RT-qPCR quantification of relative miR-21-3p level in the epidermis of acutely irradiated (Ac-UV) and non-irradiated PPARβ/δ +/+ (black) and PPARβ/δ -/- (grey) mice. (*) pvalue<0.01, N=4. (F) RT-qPCR quantification of relative miR-21-3p levels in HaCat cells treated with the PPARβ/δ agonists GW501516 or GW0742. (*) pvalue<0.01, N=3.
**Figure 2** **shows that inflammatory markers are up-regulated in miR-21-3p mimic treated HaCat keratinocytes.** Relative mRNA level of Cox2 (A) and IL6 (B) determined by real-time qPCR quantification.
**Figure 3** **shows that phosphatidylcholine content is up-regulated in miR-21-3p mimic treated HaCat keratinocytes.** Bars indicate the fold changes in mimic treated HaCat keratinocytes compared to control treated HaCat keratinocytes.
**Figure 4** **shows that the cell cycle inhibitors p15 and p21 are down regulated in miR-21-3p mimic treated HaCat keratinocytes (white) compared to control (black).**
**Figure 5** **shows that MMP1 is down regulated in miR-21-3p mimic treated HaCat keratinocytes (white) compared to control (black).**
**Figure 6** **miR-21-3p inhibitor fluorescence increases in the skin upon UV-induced inflammatory condition.** Ratio of miR-21-3p fluorescence (FAM) on control fluorescence value quantified in skin, liver and spleen of acutely irradiated (Ac-UV, white) or non-irradiated (no UV, black) mice. N=4.

### Detailed Description of the invention

The terms "Micro-RNA", "miRNA", and "miR" designate herewith a class of 17-25 nucleotides non-coding single stranded RNA molecules that regulate the expression of target RNA by either translational inhibition or mRNAs degradation. Animal miRNAs typically exhibit only partial complementarity to their mRNA targets. A 'seed region' of about 6-8 nucleotides in length at the 5' end of an animal miRNA is thought to be an important determinant of target specificity. In animals, miRNAs are generated from primary transcripts (pri-miRNAs) generally transcribed from polymerase-II (Pol-II) promoters and processed in the nucleus by Drosha enzyme to about 60-70 nucleotides stem-loop (sl) molecules with two-nucleotide 3' overhangs (pre-miRNA). Pre-miRNAs are shuttled by exportin 5 to the cytosol where Dicer enzyme releases an about 21-24 double-stranded RNA from the stem. This is loaded into the RNA-induced silencing complex (RISC), which generally selects one of the two strands as the guide strand (mature miRNA), according to thermodynamic properties. The other strand, called the passenger strand, was generally thought, up till recently, to be degraded and its function has been largely discounted. RISC targets mRNA with complementary sequence to the miRNA and downregulates their expression decreasing transcript translation and stability by a variety of molecular mechanisms.

"MicroRNA-21-3p", "miRNA-21-3p", "miR-21-3p", "microRNA-21*", "miR-21*" or "miR21*", designate the passenger strand of the pre-miRNA-21, the guide strand of said pre-mi-RNA being the miR-21-5p. The nucleic acid sequence of human miR-21-3p is represented under SEQ ID NO: 1 (miRBase Accession number MIMAT0004494). The nucleic acid sequence of murine miR-21-3p is represented under SEQ ID NO: 2 (miRBase Accession number MIMAT0004628). Human and murine miR-21-3p sequences differ only by two nucleotides. The term "miR-21-3p" covers the different variants of miR-21-3p of different mammalian species. As used herewith the term "miR-21-3p" covers the mature miR-21-3p, as well as the region, within the primiRNA-21 and pre-miRNA-21, having a nucleotide sequence identical to that of miR-21-3p.

The term "variant of a nucleic acid" as referred to herein, includes a nucleic acid (e.g. RNA or DNA) substantially homologous to the original nucleic acid sequence, but which has at least one nucleotide different from that of the original sequence because of one or more deletions, insertions or substitutions. Substantially homologous means a variant nucleic acid sequence that is at least 80%, at least 85%, at least 90%, at least 95%, at least 96%, at least 97%, at least 98% or at least 99% identical to the original nucleic acid sequence. The variant of a nucleic acid that is an inhibitor of a miR-21-3p as defined herewith is capable of inhibiting the function of said miR-21-3p.

The terms "Percentage of identity", "% identity", or the like, refer to the level of identity between two nucleic acid sequences. The percentage of identity of two sequences can be determined by visual inspection and/or mathematical calculation, or more easily by comparing sequence information using known computer program used for sequence comparison such as Clustal package version 1.83.

The term "to hybridize" or "hybridizing" means annealing nucleic acid strands from different sources, i.e. forming base pairs between complementary regions of two strands of nucleic acids that were not originally paired, for instance under stringent conditions. Typically, "hybridization under stringent conditions" as defined according to Sambrook et al. (molecular Cloning, A Laboratory Manual, Cold Spring Harbor, Laboratory Press (1989), 1.101 -1.104*)* is best obtained with fully complementary regions of two strands of nucleic acids. However, depending on various parameters such as the length of the regions of the nucleic acids to be hybridized and their specific nucleotide sequences, a few mismatches may be tolerated without substantially impairing hybridization. In particular embodiments, two nucleic acids are considered to hybridize to each other when the percentage of identity between one of the nucleic acid sequence and the complementary sequence of the other nucleic acid sequence is at least 80%, at least 85%, at least 90%, at least 95%, at least 96%, at least 97%, at least 98% or at least 99%.

The expression "Inflammatory skin disorders" as defined herewith designates disorders affecting the skin accompanied by an inflammatory process. This includes psoriasis, dermatitis, acne, rosacea, Stevens-Johnson syndrome, toxic epidermal necrolysis, systemic lupus erythematosus, skin allergies, atopic eczema, parakeratosis.

"Psoriasis" is a common, chronic immune-mediated skin disease which may also affect the joints. Psoriatic skin shows symptoms of inflammation and raised and scaly lesions. There are five main types of psoriasis: plaque, guttate, inverse, pustular, and erythrodermic. The most common form, plaque psoriasis, is commonly seen as red and white hues of scaly patches appearing on the top layer of the skin. Plaques frequently occur on the skin of the elbows and knees, but can affect any area, including the scalp, palms of hands and soles of feet, and genitals. In contrast to eczema, psoriasis is more likely to be found on the outer side of the joint. Fingernails and toenails are frequently affected (psoriatic nail dystrophy) and can be seen as an isolated sign. When psoriasis is accompanied with an inflammation of the joints, it is called "psoriatic arthritis", which is also covered by the term "psoriasis" as defined herewith. Up to 30% of individuals with psoriasis also have psoriatic arthritis.

The terms "dermatitis" or "eczema" designate herewith an inflammation of the skin. These terms broadly apply to a range of persistent skin conditions including dryness and recurring skin rashes that are characterized by one or more of the following symptoms: redness, skin swelling, itching and dryness, crusting, flaking, blistering, cracking, oozing, or bleeding. Main types of dermatitis include atopic dermatitis, contact dermatitis, xerotic eczema, seborrheic dermatitis.

The expression "skin cancers" designates cancers affecting the skin, including squamous cell carcinomas, basal cell carcinomas and melanomas.

An "inhibitor of a micro-RNA" as defined herewith designates any molecule, biologically active compound, or complex of molecules, e.g. a polypeptide, a nucleic acid, peptide nucleic acid (PNA), or a small chemical molecule, that is able to repress said micro-RNA expression and/or inhibit said micro-RNA function/activity. For instance, the function of a micro-RNA can be inhibited by a molecule such as a nucleic acid sequence complementary to the micro-RNA's seed region.

The terms "short hairpin RNA", "small hairpin RNA" and "shRNA" designate herewith a sequence of RNA that makes a tight hairpin turn that can be used to silence target gene expression via RNA interference (RNAi). Expression of shRNA in cells is typically accomplished by delivery of plasmids or through viral or bacterial vectors.

The term "small inhibitory nucleic acids" (siNAs) refers to short nucleic acids used in strategies targeting mRNA recognition and its downregulation based on their antisense action. This term covers antisense oligonucleotides, catalytic nucleic acids such as ribozymes and deoxyribozymes, as well as small interfering RNAs (siRNAs).

The term "siRNA" refers to small interfering RNA which are single or double stranded RNA (about 19-23 nucleotides) able to knock down or silence a targeted mRNA from a target gene. Artificial siRNAs can be either chemically synthesized as oligonucleotides or cloned into a plasmid or a virus vector (adenovirus, retrovirus or lentivirus) as short hairpin RNAs (shRNAs) to generate a transient or stable transfection in any type of cells (Martin et al., 2007, Ann. Rev. Genomics Hum. Genet., 8:81-108*;* Kolfschoten et al., 2007, Nat. Clin. Tract. Endocrinol. Metab., 3(12):827-34*;* Huang et al., 2008, Expect. Opin. Ther. Targets, 12(5), 637-645)*.*

As used herewith an "antagomir" or "anti-miR" designates an antisense oligonucleotide (e.g. DNA, RNA, chimeric DNA/RNA, LNA, chimeric LNA/DNA) harboring the full or partial complementary sequence of a mature miRNA that can reduce the endogenous levels of a miRNA. This term also covers antagomirs which have been chemically modified such as for instance as described in van Rooij (Circ Res. 2011, 108:219-234*).* Examples of chemical modifications include 2'-O-methyl-group (OMe)-modified oligonucleotides and locked nucleic acid (LNA)-modified oligonucleotides, in which the 2'-O-oxygen is bridged to the 4'-position through a methylene linker to form a rigid bicycle, locked into a C3'-endo (RNA) sugar conformation. Another chemical modification applied to enhance oligonucleotide stability is the balance between phosphodiester and phosphorothioate linkages between the nucleotides, with phosphorothioate providing more stability to the oligonucleotide and making it more resistant to nucleases.

A "sponge vector" typically designates a vector, in particular a lentiviral vector, for overexpressing miRNA target sequences complementary to a miRNA seed region. The nucleic acid sequence comprised in the sponge vector does not need to be perfectly complementary to the miRNA seed region for inhibiting said micro-RNA function.

The term "subject" as used herein refers to mammals. For example, mammals contemplated by the present invention include human, primates, domesticated animals such as cattle, sheep, pigs, horses, laboratory rodents and the like.

The term "LNA-modified oligonucleotide" includes any oligonucleotide either fully or partially modified with LNA monomers. Thus, an LNA-modified oligonucleotide may be composed entirely by LNA monomers, or a LNA-modified oligonucleotide may comprise one LNA monomer. As used herein, the term "LNA monomer" typically refers to a nucleoside having a 2'-4'cyclic linkage, as described in the International Patent Application WO 99/14226.

The expression "biological sample" refers to a clinical sample for testing which is taken from a mammal such as biopsy material, in particular skin samples.

The expression "control sample" refers to a negative control sample. A negative control sample includes a biological sample taken from a subject that is of the same or homologous species as the subject to be assayed for miR-21-3p and is known to have a normal biological state, e.g. without detectable symptoms of an inflammatory skin disease. A negative control sample includes a sample taken from a control subject.

As used herein, "treatment" and "treating" and the like generally mean obtaining a desired pharmacological and physiological effect. The effect may be prophylactic in terms of preventing or partially preventing a disease, symptom or condition thereof and/or may be therapeutic in terms of a partial or complete cure of a disease, condition, symptom or adverse effect attributed to the disease. The term "treatment" as used herein covers any treatment of a disease in a mammal, particularly a human, and includes: (a) preventing the disease from occurring in a subject which may be predisposed to the disease but has not yet been diagnosed as having it for example based on familial history; (b) inhibiting the disease, i.e., arresting its development; or relieving the disease, i.e., causing regression of the disease and/or its symptoms or conditions such as improvement or remediation of damage. Treatment of a skin disorder can be accompanied by a reduction in redness, itching, dryness, and/or swelling, for instance. In particular, treatment of psoriasis can be accompanied by a reduction of patches, papules or plaques on the skin. Treatment of a skin cancer can be accompanied by a decrease or eradication of the cancer stem cell populations which are at the origin of the tumor, tumor growth, recurrence and metastasis, and/or a decrease or eradication of tumor growth.

The term "efficacy" of a treatment or method according to the invention can be measured based on changes in the course of disease or condition in response to a use or a method according to the invention. For example, in the case of an inflammatory skin disorder like psoriasis, the efficacy of a treatment or method according to the invention can be measured by a reduction of patches, papules or plaques, and desquamations, on the skin, a reduction of the crisis frequency, a reduction of scratching, a reduction in the psoriasis area or/and of the Severity Index (PASI), a reduction of inflammatory, proliferation, and other specific markers (e.g. I16, PTGS2 mRNA and or proteins). For example, in the case of skin cancers, the efficacy of a treatment or method according to the invention regarding can be measured by a reduction of tumor volume, and/or an increase of progression free survival time, and/or a decreased risk of relapse post-resection for primary cancer.

The term "effective amount" as used herein refers to an amount of at least one miRNA inhibitor useful in the invention, composition or pharmaceutical formulation thereof, that elicits the biological or medicinal response in a tissue, system, animal or human that is being sought. In one embodiment, the effective amount is a "therapeutically effective amount" for the alleviation of the symptoms of the disease or condition being treated. In another embodiment, the effective amount is a "prophylactically effective amount" for prophylaxis of the symptoms of the disease or condition being prevented. The term also includes herein the amount of miRNA inhibitor sufficient to reduce the progression of the disease, notably to reduce or inhibit the symptoms of the skin disorder and thereby elicit the response being sought (i.e. an "inhibition effective amount").

### Agents and compositions

### MiR-21-3p inhibitors

In a first aspect, the invention provides inhibitors of miR-21-3p, in particular inhibitors of miR-21-3p for use as a medicament and the use of inhibitors of miR-21-3p for the preparation of a medicament, as well as compositions comprising said inhibitors.

An inhibitor of miR-21-3p is a compound which reduces or represses the activity and/or expression of miR-21-3p. In a particular embodiment, inhibitors of miR-21-3p according to the invention reduces or represses the activity and/or expression of miR-21-3p by interacting with the mature miR-21-3p, the pri-miR-21 and/or the pre-miR-21. Preferably, the inhibitor has no effect or substantially no effect on miRNAs which are not miR-21-3p.

According to the invention, inhibitors of miR-21-3p include polypeptides, nucleic acids (e.g. antisense oligonucleotides, shRNA, siRNA, antagomirs), peptide nucleic acids (PNA), or small chemical molecules, which are able to repress miR-21-3p expression and/or inhibit miR-21-3p activity.

It is understood that the variants and fragments of nucleic acids as described herewith, which constitute the miR-21-3-p inhibitors according to the invention, are able to reduce or repress the activity and/or expression of miR-21-3p.

MiR-21-3p expression can be determined by any standard method known in the art, including quantitative PCR, RT-PCR, real-time PCR, RT-LAMP, RNA sequencing, bead-based flow cytometry, microarrays, Northern blotting, dot blotting, RNase protection assays, primer extension analysis, miRNA *in situ* hybridization, and Invader(™) assays.

MiR-21-3p activity can be evaluated by determining the binding of miR-21-3p to at least one of its target(s), the assessment of de-repression of at least one of its target(s), for instance, by quantitative PCR, Western blot analysis and genome-wide transcriptional (e.g. microarray, RNA sequencing) or proteomic analyses, and miRNA reporter assays.

In one embodiment, appropriate inhibitors of miR-21-3p correspond to oligonucleotides hybridizing to:
(i) the nucleic acid sequence of miR-21-3p or a fragment thereof, in particular human miR-21-3p of SEQ ID NO: 1 or murine miR-21-3p of SEQ ID NO: 2 or a variant thereof or a fragment thereof, and/or
(ii) the seed region of miR-21-3p, such as a sequence corresponding to nucleotides 2 to 7 of SEQ ID NO: 1 or nucleotides 2 to 7 of SEQ ID NO: 2 or a variant thereof, and/or
(iii) a fragment of at least 10 contiguous nucleotides from the nucleic acid sequence of miR-21-3p, such as human miR-21-3p of SEQ ID NO: 1 or murine miR-21-3p of SEQ ID NO: 2 or a variant thereof, optionally comprising the seed region of miR-21-3p, such as a sequence corresponding to nucleotides 2 to 7 of SEQ ID NO: 1 or nucleotides 2 to 7 of SEQ ID NO: 2 or a variant thereof.

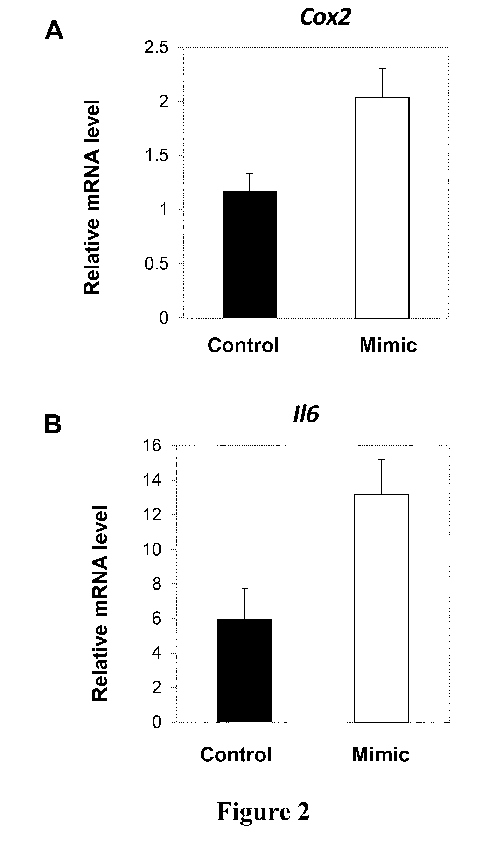

It is believed that the seed region of mammalian miR-21-3p extends from nucleotides 2 to 7 of mature miR-21-3p sequence (Figure 2 *of* Lo et al, Plos One, 2013, 8(9):1-11)*.* For instance, the seed region of human miR-21-3p could correspond to nucleotides 2 to 7 of SEQ ID NO: 1, i.e. AACACC, the seed region of murine miR-21-3p could correspond to nucleotides 2 to 7 of SEQ ID NO: 2, i.e. AACAGC.

Typically, a fragment of the nucleic acid sequence of miR-21-3p that hybridizes with the miR-21-3p inhibitors according to the invention may have about 5 to 21 nucleotides, in particular about 5, 6, 7, 8, 9 or 10 to 21 nucleotides, more particularly about 20 nucleotides e.g. 15-21 nucleotides, for example about 15, 16, 17, 18, 19, 20, or 21 nucleotides.

Appropriate miR-21-3p inhibitors may include single or double stranded oligonucleotides which are able to bind to mature miR-21-3p, in particular to the seed region of mature miR-21-3p, or to the passenger strand of miR-21 precursors, and inhibit the activity of mature miR-21-3p, prevent or inhibit its production and/or increase its rate of depletion.

Appropriate oligonucleotides may be oligodeoxyribonucleotides, oligoribonucleotides or modified oligonucleotides as described below.

In one embodiment of the invention, an inhibitor of miR-21-3p is a single stranded oligonucleotide which has a sequence which is sufficiently complementary to the sequence of miR-21-3p to hybridize to said miR-21-3p by Watson-Crick base-pairing. In a particular embodiment, an inhibitor of miR-21-3p comprises a single stranded oligonucleotide complementary to miR-21-3p nucleic acid sequence and/or complementary to a fragment of miR-21-3p, said fragment reducing or repressing the activity and/or expression of miR-21-3p.

In a particular embodiment, an inhibitor of miR-21-3p comprises a single stranded oligonucleotide complementary to miR-21-3p nucleic acid sequence and/or complementary to a fragment of miR-21-3p, said fragment comprising the seed region of miR-21-3p.

In another particular embodiment, said fragment comprises at least 6, 7, 8, 9, or at least 10 nucleotides.

In another embodiment, an inhibitor of miR-21-3p comprises a single stranded oligonucleotide, hybridizing to:
(i) the nucleic acid sequence of miR-21-3p or a fragment thereof, such as human miR-21-3p of SEQ ID NO: 1 or murine miR-21-3p of SEQ ID NO: 2 or a variant thereof or a fragment thereof, and/or
(ii) the seed region of miR-21-3p, in particular a sequence corresponding to nucleotides 2 to 7 of SEQ ID NO: 1 or nucleotides 2 to 7 of SEQ ID NO: 2 or a variant thereof, and/or
(iii) a fragment of at least 10 contiguous nucleotides from the nucleic acid sequence of miR-21-3p, in particular human miR-21-3p of SEQ ID NO: 1 or murine miR-21-3p of SEQ ID NO: 2 or a variant thereof, optionally comprising the seed region of miR-21-3p, in particular a sequence corresponding to nucleotides 2 to 7 of SEQ ID NO: 1 or nucleotides 2 to 7 of SEQ ID NO: 2 or a variant thereof.

In a further embodiment, an inhibitor of miR-21-3p is an oligonucleotide, in particular a single stranded oligonucleotide, comprising SEQ ID NO: 3, or a variant thereof, or a fragment thereof able to reduce or repress the activity and/or expression of miR-21-3p. In a further embodiment, an inhibitor of miR-21-3p is an oligonucleotide, in particular a single stranded oligonucleotide, comprising SEQ ID NO: 3, or a variant thereof, or a fragment thereof comprising nucleotides 15 to 20 of SEQ ID NO: 3.

In a more particular embodiment, said fragment comprises at least 6, 7, 8, 9, 10, 11, 12, 13, 14, 15, 16, 17, 18, 19, or 20 contiguous nucleotides from SEQ ID NO: 3.

In a particular embodiment, an inhibitor of miR-21-3p is an oligonucleotide, in particular a single stranded oligonucleotide, comprising a fragment of at least 10 contiguous nucleotides from SEQ ID NO: 3.

In a particular embodiment, an inhibitor of miR-21-3p is an oligonucleotide, in particular a single stranded oligonucleotide, comprising a fragment of at least 10 contiguous nucleotides from SEQ ID NO: 3, or a variant thereof, and comprising nucleotides 15 to 20 of SEQ ID NO: 3.

In a further particular embodiment, an inhibitor of miR-21-3p is an oligonucleotide, in particular a single stranded oligonucleotide, comprising SEQ ID NO: 4 or SEQ ID NO: 5.

In a further embodiment, an inhibitor of miR-21-3p is an oligonucleotide, in particular a single stranded oligonucleotide, comprising SEQ ID NO: 6, or a variant thereof, or a fragment thereof able to reduce or repress the activity and/or expression of miR-21-3p. In a further embodiment, an inhibitor of miR-21-3p is an oligonucleotide, in particular a single stranded oligonucleotide, comprising SEQ ID NO: 6, or a variant thereof, or a fragment thereof comprising nucleotides 15 to 20 of SEQ ID NO: 6.

In a more particular embodiment, said fragment comprises at least 6, 7, 8, 9, 10, 11, 12, 13, 14, 15, 16, 17, 18, 19, or 20 contiguous nucleotides from SEQ ID NO: 6.

In a particular embodiment, an inhibitor of miR-21-3p is an oligonucleotide, in particular a single stranded oligonucleotide, comprising a fragment of at least 10 contiguous nucleotides from SEQ ID NO: 6, or a variant thereof.

In a particular embodiment, an inhibitor of miR-21-3p is an oligonucleotide, in particular a single stranded oligonucleotide, comprising a fragment of at least 10 contiguous nucleotides from SEQ ID NO: 6, or a variant thereof, and comprising nucleotides 15 to 20 of SEQ ID NO: 6.

In a further particular embodiment, an inhibitor of miR-21-3p is an oligonucleotide, in particular a single stranded oligonucleotide, comprising SEQ ID NO: 7 or SEQ ID NO: 8.

Typically, an appropriate oligonucleotide for inhibition of miR-21-3p may have about 6 to 30 nucleotides, in particular about 10 to 30 nucleotides, more particularly about 20 nucleotides e.g. 15-21 nucleotides, for example about 15, 16, 17, 18, 19, 20, or 21 nucleotides.

As will be understood by one skilled in the art, when the inhibitor of miR-21-3p is an RNA molecule, said inhibitor may comprise any one of the above-mentioned sequences, except that the five-carbon pentose of the backbone is a ribose instead of a deoxyribose, and the nitrogenous base uracil (U) replaces the thymine (T).

In another embodiment, the inhibitor of miR-21-3p corresponds to a double stranded DNA placed under the control of a promoter that allows expression of a transcript complementary to miR-21-3p, or complementary to a fragment of miR-21-3p optionally comprising the miR-21-3p seed region, as for example in a sponge vector. Thus, the sequence comprised in the sponge vector can be identical to the seed region of miR-21-3p seed region except for a few (e.g. about 1, 2, 3) different nucleotides. The exact number of different nucleotides are determined empirically for each miRNA, by the skilled person.

In particular embodiments, the oligonucleotides for the inhibition of miR-21-3p activity are chemically modified.

The chemical modifications of said oligonucleotides may comprise modifications to the nucleobases, the backbone residues, and/or the internucleoside linkers of said oligonucleotides.

Modifications to one or more nucleobases of said oligonucleotides may comprise one or more alkylated purines and pyrimidines, acylated purines and pyrimidines, and other heterocycles. These classes of pyrimidines and purines are known in the art and include pseudoisocytosine, N4,N4-ethanocytosine, 8-hydroxy-N-6-methyladenine, 4-acetylcytosine, 5-(carboxyhydroxylmethyl)uracil, 5 fluorouracil, 5-bromouracil, 5-carboxymethylaminomethyl-2-thiouracil, 5-carboxymethylaminomethyl uracil, dihydrouracil, inosine, N6-isopentyl-adenine, 1-methyladenine, 1-methylpseudouracil, 1-methylguanine, 2,2-dimethylguanine, 2-methyladenine, 2-methylguanine, 3-methylcytosine, 5-methylcytosine, N6-methyladenine, 7-methylguanine, 5-methylaminomethyl uracil, 5-methoxy amino methyl-2-thiouracil, -D-mannosylqueosine, 5-methoxycarbonylmethyluracil, 5-methoxyuracil, 2 methylthio-N-6-isopentenyladenine, uracil-5-oxyacetic acid methyl ester, psueouracil, 2-thiocytosine, 5-methyl-2 thiouracil, 2-thiouracil, 4-thiouracil, 5-methyluracil, N-uracil-5-oxyacetic acid methylester, uracil 5-oxyacetic acid, queosine, 2-thiocytosine, 5-propyluracil, 5-propylcytosine, 5-ethyluracil, 5-ethylcytosine, 5-butyluracil, 5-pentyluracil, 5-pentylcytosine, and 2,6,diaminopurine, methylpsuedouracil, 1-methylguanine and 1-methylcytosine.

Modifications to one or more backbone residues of said oligonucleotides may comprise one or more of the following: 2' sugar modifications such as 2'-O-methyl (2'-OMe), 2'-O-methoxyethyl (2'-MOE), 2'-O-methoxyethoxy, 2'-Fluoro (2'-F), 2'-Allyl, 2'-O-[2-(methylamino)-2-oxoethyl], 2'-O-(N-methylcarbamate); 4' sugar modifications including 4'-thio, 4'-CH₂-O-2'-bridge, 4-(CH₂)₂-O-2'-bridge; Locked Nucleic Acid (LNA); Peptide Nucleic Acid (PNA); Intercalating nucleic acid (INA); Twisted intercalating nucleic acid (TINA); Hexitol nucleic acids (HNA); arabinonucleic acid (ANA); cyclohexane nucleic acids (CNA); cyclohexenylnucleic acid (CeNA); threosyl nucleic acid (TNA); Morpholino oligonucleotides; Gap-mers; Mix-mers; Incorporation Arginine-rich peptides; addition of 5'-phosphate to synthetic RNAs; RNA Aptamers; or any combinations thereof.

Modifications to one or more internucleoside linkers of said oligonucleotides may comprise one or more of the following: Phosphorothioate, phosphoramidate, phosphorodiamidate, phosphorodithioate, phosphoroselenoate, phosphorodiselenoate, phosphoroanilothioate and phosphoranilidate, or any combinations thereof.

Locked Nucleic Acid (LNA) nucleosides are a class of nucleic acid analogues in which the ribose ring is "locked" by a methylene bridge connecting the 2'-O atom and the 4'-C atom. LNA nucleosides contain the common nucleobases that appear in DNA and RNA and are able to form base pairs according to standard Watson-Crick base pairing rules. However, by "locking" the molecule with the methylene bridge the LNA is constrained in the ideal conformation for Watson-Crick binding. When LNA nucleosides are incorporated into a DNA or RNA oligonucleotide, this makes the pairing with a complementary nucleotide strand more rapid and increases the stability of the resulting duplex. This increased stability, as well as the high hybridization affinity and improved mismatch discrimination abilities, make LNA-modified oligonucleotides extremely potent antisense inhibitors, both for *in vitro* and *in vivo* use. When transfected in the cells, LNA-modified oligonucleotides display high nuclease resistance and low cytotoxicity. It is also possible to further modify LNA-modified oligonucleotides to include a phosphorothioate backbone, which further increases nuclease resistance of the oligonucleotide and improves the efficiency of the inhibition.

An LNA-modified oligonucleotide contains one or more units of an LNA monomer, preferably one or more 2'-O, 4'-C-methylene bridge monomers (oxy-LNA). An LNA-modified oligonucleotide however also may contain other LNA units in addition to or in place of an oxy-LNA group. In particular, preferred additional LNA units include 2'-thio-LNA (thio-LNA), 2'-HN-LNA (amino-LNA), and 2'-N (R)-LNA (amino-R-LNA)) monomers in either the D-B or L-a configurations or combinations thereof.

An LNA-modified oligonucleotide also may have other internucleoside linkages than the native phosphodiester, e.g. phosphoromonothioate, phosphorodithioate, and methylphosphonate linkages. The LNA-modified oligonucleotide can be fully modified with LNA (i.e. each nucleotide is an LNA unit), but it is generally preferred that the LNA-modified oligomers will contain other residues such as native DNA monomers, phosphoromonothioate monomers, methylphosphonate monomers or analogs thereof. In general, an LNA-modified oligonucleotide will contain at least about 5, 10, 15 or 20 percent LNA units, based on total nucleotides of the oligonucleotide, more typically at least about 20, 25, 30, 40, 50, 60, 70, 80 or 90 percent LNA units, based on total bases of the oligonucleotide.

In a particular embodiment, the inhibitor of miR-21-3p is a LNA-modified oligonucleotide.

In a still further embodiment, the inhibitor of miR-21-3p is a modified LNA-oligonucleotide hybridizing to:
(i) the nucleic acid sequence of miR-21-3p or a fragment thereof, in particular human miR-21-3p of SEQ ID NO: 1 or murine miR-21-3p of SEQ ID NO: 2 or a variant thereof, or a fragment thereof, and/or
(ii) the seed region of miR-21-3p, in particular a sequence corresponding to nucleotides 2 to 7 of SEQ ID NO: 1 or nucleotides 2 to 7 of SEQ ID NO: 2 or a variant thereof, and/or
(iii) a fragment of at least 10 contiguous nucleotides from the nucleic acid sequence of miR-21-3p, in particular human miR-21-3p of SEQ ID NO: 1 or murine miR-21-3p of SEQ ID NO: 2 or a variant thereof, optionally comprising the seed region of miR-21-3p, such as a sequence corresponding to nucleotides 2 to 7 of SEQ ID NO: 1 or nucleotides 2 to 7 of SEQ ID NO: 2 or a variant thereof,
wherein the ribose ring of one or more nucleotides of said oligonucleotide is locked by a methylene bridge connecting the 2'-O atom and the 4'-C atom.

In a more particular embodiment, the inhibitor of miR-21-3p is a LNA-modified oligonucleotide comprising SEQ ID NO: 3, or a variant thereof, or a fragment thereof, wherein the ribose ring of one or more nucleotides of said oligonucleotide is locked by a methylene bridge connecting the 2'-O atom and the 4'-C atom.

In a more particular embodiment, the inhibitor of miR-21-3p is a LNA-modified oligonucleotide comprising SEQ ID NO: 3, or a variant thereof, or a fragment thereof comprising nucleotides 15 to 20 of SEQ ID NO: 3, wherein the ribose ring of one or more nucleotides of said oligonucleotide is locked by a methylene bridge connecting the 2'-O atom and the 4'-C atom.

Still more particularly, said fragment comprises at least 6, 7, 8, 9, 10, 11, 12, 13, 14, 15, 16, 17, 18, 19, or 20 contiguous nucleotides from SEQ ID NO: 3.

In a particular embodiment, the inhibitor of miR-21-3p is a LNA-modified oligonucleotide comprising a fragment of at least 10 contiguous nucleotides from SEQ ID NO: 3, or a variant thereof.

In a particular embodiment, the inhibitor of miR-21-3p is a LNA-modified oligonucleotide comprising a fragment of at least 10 contiguous nucleotides from SEQ ID NO: 3, or a variant thereof, comprising nucleotides 15 to 20 of SEQ ID NO: 3, wherein the ribose ring of one or more nucleotides of said oligonucleotide is locked by a methylene bridge connecting the 2'-O atom and the 4'-C atom.

In a still more particular embodiment, the inhibitor of miR-21-3p is a LNA-modified oligonucleotide comprising SEQ ID NO: 4 or SEQ ID NO: 5, wherein the ribose ring of one or more nucleotides of said oligonucleotide is locked by a methylene bridge connecting the 2'-O atom and the 4'-C atom.

In a further embodiment, an inhibitor of miR-21-3p is a LNA-modified oligonucleotide comprising SEQ ID NO: 6, or a variant thereof, or a fragment thereof, wherein the ribose ring of one or more nucleotides of said oligonucleotide is locked by a methylene bridge connecting the 2'-O atom and the 4'-C atom.

In a further embodiment, an inhibitor of miR-21-3p is a LNA-modified oligonucleotide comprising SEQ ID NO: 6, or a variant thereof, or a fragment thereof comprising nucleotides 15 to 20 of SEQ ID NO: 6, wherein the ribose ring of one or more nucleotides of said oligonucleotide is locked by a methylene bridge connecting the 2'-O atom and the 4'-C atom.

More particularly, said fragment comprises at least 6, 7, 8, 9, 10, 11, 12, 13, 14, 15, 16, 17, 18, 19, or 20 contiguous nucleotides from SEQ ID NO: 6.

In a particular embodiment, the inhibitor of miR-21-3p is a LNA-modified oligonucleotide comprising a fragment of at least 10 contiguous nucleotides from SEQ ID NO: 6, or a variant thereof, wherein the ribose ring of one or more nucleotides of said oligonucleotide is locked by a methylene bridge connecting the 2'-O atom and the 4'-C atom.

In a particular embodiment, the inhibitor of miR-21-3p is a LNA-modified oligonucleotide comprising a fragment of at least 10 contiguous nucleotides from SEQ ID NO: 6, or a variant thereof, comprising nucleotides 15 to 20 of SEQ ID NO: 6, wherein the ribose ring of one or more nucleotides of said oligonucleotide is locked by a methylene bridge connecting the 2'-O atom and the 4'-C atom.

In a further embodiment, an inhibitor of miR-21-3p is a LNA-modified oligonucleotide comprising SEQ ID NO: 7 or SEQ ID NO: 8, wherein the ribose ring of one or more nucleotides of said oligonucleotide is locked by a methylene bridge connecting the 2'-O atom and the 4'-C atom.

In another particular embodiment, the inhibitor of miR-21-3p is an antagomir or a chemically modified antagomir including, for instance, 2' methoxi groups and phosphothioates. Production of antagomirs may be achieved for instance as disclosed in Krutzfeldt et al (2005, Nature, 438, 685-689*).*

Modified oligonucleotides may also contain one or more sugar mimetics instead of a pentofuranosyl sugar. Suitable sugar mimetics include cyclobutyl moieties, azido-ribose, carbocyclic sugar analogues a-anomeric sugars; epimeric sugars such as arabinose, xyloses or lyxoses, pyranose sugars, furanose sugars, and sedoheptulose.

In some embodiments, both the sugar and the backbone linkage of one or more, preferably all of the nucleotides in a modified oligonucleotide may be replaced with non-natural groups. The bases are maintained for hybridization with miR-21-3p. Suitable modified oligonucleotides may include peptide nucleic acids (PNA). In PNA, the oligonucleotide sugar-backbone is replaced with an amide containing backbone, in particular an aminoethylglycine backbone. The bases are retained and are bound directly or indirectly to aza-nitrogen atoms of the amide portion of the backbone.

In a further embodiment, modified oligonucleotides may be chemically linked to one or more moieties or groups which enhance the activity, cellular distribution or cellular uptake of the oligonucleotide. Suitable moieties include lipid moieties such as cholesterol, cholic acid, a thioether, e.g., hexyl-S-tritylthiol, a thiocholesterol, an aliphatic chain, e.g., dodecandiol or undecyl residues, a phospholipid, e.g., di-hexadecyl-rac-glycerol or triethyl-ammonium 1,2-di-O-hexadecyl-rac-glycero-3-H-phosphonate, a polyamine or a polyethylene glycol chain, or adamantane acetic acid, a palmityl moiety, or an octadecylamine or hexylamino-carbonyl-oxycholesterol moiety. In a particular embodiment, said oligonucleotides and/or modified oligonucleotides are chemically linked to cholesterol.

In another embodiment, the inhibitor of miR-21-3p is a polypeptide or a peptide that is able to repress said micro-RNA expression and/or inhibit said micro-RNA function/activity.

In another embodiment, the inhibitor of miR-21-3p is a peptide nucleic acid (PNA) that is able to repress said micro-RNA expression and/or inhibit said micro-RNA function/activity.

In another embodiment, the inhibitor of miR-21-3p is a small chemical molecule that is able to repress said micro-RNA expression and/or inhibit said micro-RNA function/activity.

### Compositions

In another embodiment, the invention relates to a composition comprising a miR-21-3p inhibitor as described above or a vector comprising a nucleic acid encoding said miR-21-3p inhibitor.

In a particular embodiment, it is provided a cosmetic composition comprising a miR-21-3p inhibitor as described above or a vector comprising a nucleic acid encoding said miR-21-3p inhibitor.

In an alternative embodiment, it is provided the use of said cosmetic composition for improving skin appearance and/or skin comfort, e.g. for increasing or restoring skin suppleness, smoothness, and/or improving or restoring skin homeostasis.

In another embodiment, the invention relates to a pharmaceutical composition comprising a miR-21-3p inhibitor as described above or a vector comprising a nucleic acid encoding said miR-21-3p inhibitor; and a pharmaceutically acceptable carrier.

The invention provides pharmaceutical or therapeutic agents as compositions and methods for treating a subject, preferably a mammalian subject, and most preferably a human patient who is suffering from a medical disorder, in particular a skin disorder, in particular a skin disorder selected from inflammatory skin disorders such as psoriasis, dermatitis, acne, rosacea, Stevens-Johnson syndrome, toxic epidermal necrolysis, systemic lupus erythematosus, skin allergies, atopic eczema, parakeratosis, keratosis, skin cancers such as squamous cell carcinoma, basal cell carcinomas and melanomas, leprosy, vitiligo, epidermolytic ichthyosis, UV photodamages, topical allergy, UV erythema, skin ageing, and wounds.

Compositions or formulations according to the invention may be administered as a pharmaceutical formulation which can contain one or more agents according to the invention in any form described herein.

The compositions according to the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous) use by injection or continuous infusion. Injectable compositions are typically based upon injectable sterile saline or phosphate-buffered saline or other injectable carriers known in the art. Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed. Compositions of this invention may be liquid formulations including, but not limited to, aqueous or oily suspensions, solutions, emulsions, syrups, and elixirs. The compositions may also be formulated as a dry product for reconstitution with water or other suitable vehicle before use. Such liquid preparations may contain additives including, but not limited to, suspending agents, emulsifying agents, non-aqueous vehicles and preservatives. Suspending agents include, but are not limited to, sorbitol syrup, methyl cellulose, glucose/sugar syrup, gelatin, hydroxyethyl cellulose, carboxymethyl cellulose, aluminum stearate gel, and hydrogenated edible fats. Emulsifying agents include, but are not limited to, lecithin, sorbitan monooleate, and acacia. Preservatives include, but are not limited to, methyl or propyl p-hydroxybenzoate and sorbic acid. Dispersing or wetting agents include but are not limited to poly(ethylene glycol), glycerol, bovine serum albumin, Tween®, Span®.

Compositions of this invention may also be formulated as a depot preparation, which may be administered by implantation or by intramuscular injection.

Solid compositions of this invention may be in the form of tablets or lozenges formulated in a conventional manner.

The compounds of this invention can also be administered in sustained release forms or from sustained release drug delivery systems.

According to a particular embodiment, compositions according to the invention are for topical use.

In a particular embodiment, the topical compositions of the present invention may also include one or more of the following: a skin penetration enhancer, an emollient, such as isopropyl myristate, petrolatum, silicones (e.g., methicone, dimethicone), oils, mineral oils, and fatty acid esters; a humectant, such as glycerin or caprylyl glycol, a skin plumper, such as palmitoyl oligopeptide, collagen, or collagen and/or glycosaminoglycan (GAG) enhancing agents, a sunscreen, such as avobenzone, an exfoliating agent, and an antioxidant. Suitable exfoliating agents include, for example, alpha-hydroxyacids, beta-hydroxyacids, oxa-acids, oxadiacids, and their derivatives such as esters, anhydrides and salts thereof. Suitable hydroxy acids include, for example, glycolic acid, lactic acid, malic acid, tartaric acid, citric acid, 2-hydroxyalkanoic acid, mandelic acid, salicylic acid and derivatives thereof. A convenient exfoliating agent is glycolic acid. When present, the exfoliating agent may comprise from about 0.1 wt % to about 80 wt % of the composition.

Examples of antioxidants that may be used in the present compositions include compounds having phenolic hydroxy functions, such as ascorbic acid and its derivatives/esters; beta-carotene; catechins; curcumin; ferulic acid derivatives (e.g. ethyl ferulate, sodium ferulate); gallic acid derivatives (e.g., propyl gallate); lycopene; reductic acid; rosmarinic acid; tannic acid; tetrahydrocurcumin; tocopherol and its derivatives; uric acid; or any mixtures thereof. Other suitable antioxidants are those that have one or more thiol functions (-SH), in either reduced or non-reduced form, such as glutathione, lipoic acid, thioglycolic acid, and other sulfhydryl compounds. The antioxidant may be inorganic, such as bisulfites, metabisulfites, sulfites, or other inorganic salts and acids containing sulfur. Compositions of the present invention may comprise an antioxidant preferably from about 0.001 wt % to about 10 wt %, and more preferably from about 0.01 wt % to about 5 wt %, of the total weight of the composition.

Other conventional additives include: vitamins, such as tocopherol and ascorbic acid; vitamin derivatives such as ascorbyl monopalmitate; thickeners such as hydroxyalkyl cellulose; gelling agents; structuring agents, metal chelating agents such as EDTA; pigments; colorants, and pH adjusters. The composition may optionally comprise other components known to those skilled in the art including, but not limited to, film formers, moisturizers, minerals, viscosity and/or rheology modifiers, anti-acne agents, insect repellents, skin cooling compounds, skin protectants, lubricants, fragrances, preservatives, stabilizers, and mixtures thereof. In addition to the foregoing, the cosmetic compositions of the invention may contain any other compound for the treatment of skin disorders.

Particularly in case of topical administration, the composition may be formulated in a variety of product forms, such as an emulsion, lotion, cream, serum, spray, aerosol, cake, ointment, essence, gel, paste, patch, pencil, towelette, mask, stick, foam, elixir, concentrate, and the like. In particular, the composition is formulated as an emulsion, lotion, cream, ointment, serum or gel.

In a further particular embodiment, the miR-21-3p inhibitor of the invention is comprised in a diphenylcyclopropenone (DPCP) gel formulation. An example of such DPCP gel formulation is described in Hagen et al (2013, J Drugs Dermatol, 12(2): 152).

In a still further embodiment, the miR-21-3p inhibitor of the invention is comprised in a poloxamere based formulation.

In a particular embodiment, the miR-21-3p inhibitor of the invention is comprised in an alcohol or water based formulation.

In another particular aspect, the compositions according to the invention are adapted for delivery by repeated administration.

Further materials as well as formulation processing techniques and the like are set out in Part 5 of Remington's "The Science and Practice of Pharmacy", 22nd Edition, 2012, University of the Sciences in Philadelphia, Lippincott Williams & Wilkins*,* which is incorporated herein by reference.

When the agents and compositions according to the invention comprise nucleic acids as described herewith, those can be prepared by incorporating the nucleic acid as an active ingredient together with a base which is commonly used for an agent for gene therapy. When nucleic acids are incorporated into a virus vector, virus particles containing recombinant vectors are prepared, and the resultants are incorporated with a base which is commonly used for an agent for gene therapy.

As a base used for incorporating the nucleic acid as an active ingredient, a base that is commonly used for an injection can be used. Examples include distilled water, a salt solution of sodium chloride or a mixture of sodium chloride and an inorganic salt, a solution of mannitol, lactose, dextran, or glucose, an amino acid solution of glycine or arginine, an organic acid solution, and a mixed solution of a salt solution and a glucose solution. Alternatively, an adjuvant, such as a regulator of osmotic pressure, a pH adjuster, vegetable oil, or a surfactant, may be added to the base in accordance with a technique known in the art to prepare an injection in the form of a solution, suspension, or dispersion. Such injection can be prepared in the form of a preparation to-be-dissolved before use via operations, such as pulverization or lyophilization. Formulations for topical administration of cosmetic compositions comprising the miR-21-3p inhibitors according to the invention can include lipid, water or alcohol base formulations like cream or shampoo.

### Uses and methods according to the invention in therapy

In another aspect of the invention, it is provided a miR-21-3p inhibitor as described herewith for use in the prevention and/or treatment of skin disorders, in particular inflammatory skin disorders, keratosis, and skin cancers.

In an alternative aspect of the invention is provided the use of a miR-21-3p inhibitor as described herewith for the manufacture of a medicament for the prevention and/or treatment of skin disorders, in particular inflammatory skin disorders, keratosis, and skin cancers.

In a still alternative aspect of the invention is provides a method of preventing and/or treating skin disorders, in particular inflammatory skin disorders, keratosis, and skin cancers, said method comprising administering in a subject in need thereof a miR-21-3p inhibitor as described herewith, or a pharmaceutical formulation thereof.

In a particular embodiment of the above-mentioned aspects of the invention, said skin disorder is selected from inflammatory skin disorders (including psoriasis, dermatitis, acne, rosacea, Stevens-Johnson syndrome, toxic epidermal necrolysis, systemic lupus erythematosus, skin allergies, atopic eczema, parakeratosis), keratosis, skin cancers (including squamous cell carcinoma, basal cell carcinomas and melanomas), leprosy, vitiligo, epidermolytic ichthyosis, UV photodamages, topical allergy, UV erythema, skin ageing, and wounds.

In a still more particular embodiment, said skin disorder is an inflammatory skin disorder, particularly an inflammatory skin disorder selected from psoriasis, dermatitis, acne, rosacea, Stevens-Johnson syndrome, toxic epidermal necrolysis, systemic lupus erythematosus, skin allergies, atopic eczema, parakeratosis.

In a still more particular embodiment, said skin disorder is psoriasis or dermatitis, more particularly psoriasis.

In a still more particular embodiment, said skin disorder is selected from keratosis or skin cancers.

In a still more particular embodiment, said skin cancer is selected among squamous cell carcinomas, basal cell carcinomas and melanomas, more particularly squamous cell carcinoma.

### Mode of administration

Compositions of this invention may be administered in any manner including intravenous injection, intra-arterial, intraperitoneal injection, subcutaneous injection, intramuscular, intra-thecal, oral route, cutaneous application, direct tissue perfusion during surgery or combinations thereof.

Considering the easy accessibility of skin, topical delivery could reveal particularly appropriate for the oligonucleotides-based treatment of disorders of this tissue, which would have the obvious advantage that doses of oligonucleotides according to the invention required for an effect are considerably lower. In addition, topical administration also allows a more focused delivery, avoiding any undesirable side-effect potentially associated with systemic delivery.

When the agents and compositions according to the invention comprise oligonucleotides or vectors as described herewith, as active ingredients, those can be administered via, for example, simple direct injection of the nucleic acid molecule, the use of carriers of genetic material (adenoviruses, lentiviral vectors, adeno associated viruses), injection of conjugates including liposomes (vesicles consisting of an aqueous compartment enclosed in a phospholipid bilayer with the drug typically entrapped in the center aqueous layer), lipoplexes (spontaneously formed with the interaction of cationic lipids and negatively charged nucleic acids, are preferably prepared immediately before use), complexes with cholesterol, peptides (e.g. signal peptide for targeting specific cells), polymers or antibodies, and chemically modified oligonucleotides. Another delivery strategy includes the use of atelocollagen (e.g. obtained from type I collagen of calf dermis by pepsin treatment) whose surface is positively charged and the molecule can bond electrostatically with negatively charged nucleic acid molecules.

Successful local siRNA delivery relevant for dermatology has been described in mice *employing in vivo* skin electroporation (Inoue et al., 2007, J Gene Med 2007; 9:994-1001*),* which could also be employed for administration of the miR-21-3p inhibitors described herewith.

### Combination

According to the invention, the agents and compositions according to the invention, and pharmaceutical formulations thereof can be administered alone or in combination with a co-agent useful in the treatment of a skin disorder.

The invention encompasses the administration of an agent or composition according to the invention and pharmaceutical formulations thereof, wherein said agent or composition is administered to an individual prior to, simultaneously or sequentially with other therapeutic regimens or co-agents useful in the treatment of a skin disorder, in particular skin inflammatory disorders, keratosis, and skin cancers, in a therapeutically effective amount.

An agent or composition according to the invention, or the pharmaceutical formulation thereof, that is administered simultaneously with said co-agents can be administered in the same or different composition(s) and by the same or different route(s) of administration.

According to one embodiment, is provided a pharmaceutical formulation comprising an agent or composition according to the invention, combined with at least one co-agent useful in the treatment of a skin disorder, preferably inflammatory skin disorders, keratosis and skin cancers, and at least one pharmaceutically acceptable carrier.

Said co-agents useful in the treatment of a skin disorder include topical retinoids (e.g. tazarotene), calcineurin inhibitors (e.g. tacrolimus and pimecrolimus), salicylic acid, coal tar, phototherapy, topical corticosteroids, vitamin D analogues, anthralin, acitretin, methotrexate, ciclosporin, mycophenolate mofetil, interferon gamma, biological agents such as alefacept, etanercept, adalimumab, efalizumab and infliximab, phototherapy, chemophototherapy, for instance, for the treatment of inflammatory skin disorders like psoriasis.

Said co-agents useful in the treatment of a skin cancer include imiquimod, radiation therapy and topical chemotherapy.

### Subjects

In an embodiment, subj ects according to the invention are subj ects suffering from a skin disorder.

In a particular embodiment, subjects according to the invention are subjects suffering from inflammatory skin disorders, more particularly from psoriasis, dermatitis, acne, rosacea, Stevens-Johnson syndrome, toxic epidermal necrolysis, systemic lupus erythematosus, skin allergies, atopic eczema, parakeratosis.

In another particular embodiment, subjects according to the invention are subjects suffering from keratosis or skin cancers, in particular selected from squamous cell carcinomas, basal cell carcinomas and melanomas, more particularly squamous cell carcinoma.

In a still other particular embodiment, subjects according to the invention are subjects suffering from leprosy, vitiligo, epidermolytic ichthyosis, UV photodamages, topical allergy, UV erythema, skin ageing, and wounds.

### Uses and methods according to the invention in diagnostics

In another aspect of the invention is provided an *ex-vivo* method of prognosis and/or diagnosis of a skin disorder in a subject comprising determining, in a biological sample of said subject, the level of miR-21-3p.

In a particular embodiment, said *ex-vivo* method of prognosis and/or diagnosis of a skin disorder in a subject comprises:
a) Providing a biological sample from a subject;
b) Determining the level of miR-21-3p, in said sample;
c) Comparing the level of miR-21-3p determined in step b) with the level of miR-21-3p in a control sample:
wherein an increased level of miR-21-3p determined in step b) compared to the control sample is indicative of the subject being at risk for developing, or having, a skin disorder.

In the method mentioned herewith, the level of miR-21-3p can be determined by any method known in the art including, for example, quantitative PCR, RT-PCR, real-time PCR, RT-LAMP, RNA sequencing, bead-based flow cytometry, microarrays, Northern blotting, dot blotting, RNase protection assays, primer extension analysis, miRNA *in situ* hybridization, and Invader(™) assays.

In the *ex-vivo* method of prognosis and/or diagnosis of a skin disease according to the invention, the biological sample is a skin sample. Said biological sample can comprise cells taken from a lesion or other site on the skin of the subject.

Appropriate control samples include cells from healthy (i.e. non-lesional) skin which is not affected by a skin disorder or skin affected with the same disorder but with an inferior severity degree. Control cells may also be obtained from a different subject than the one from whom the biological sample has been extracted and who is submitted to the test, for example a healthy individual not suffering from, or susceptible to, a skin disorder or suffering of the same disorder but with an inferior severity degree than the tested subject.

References cited herein are hereby incorporated by reference in their entirety. The present invention is not to be limited in scope by the specific embodiments described herein, which are intended as single illustrations of individual aspects of the invention, and functionally equivalent methods and components are within the scope of the invention. Indeed, various modifications of the invention, in addition to those shown and described herein will become apparent to those skilled in the art from the foregoing description and accompanying drawings. Such modifications are intended to fall within the scope of the appended claims.

The invention having been described, the following examples are presented by way of illustration, and not limitation.

### EXAMPLES

### Materials and Methods

### - Animal model, acute and chronic UV irradiation, GSK0660 treatments

Hairless PPARβ/δ +/+ and PPARβ/δ -/- mice (Leibowitz et al. 2000, FEBS Lett. 473, 333-336*)* were housed in quarters with 12/12h light/dark cycle and maintained with water and food ad libitum. UV irradiation of at least four mice per group was performed using a solar UV lamp (UV DUKE GL40E 40Watts, Griot SA, UVB/UVA<0.1%). For acute UV exposure, 9-12 weeks old females were irradiated on their backs, and UV radiation emission was controlled using a radiometer until a dose of 120mJ/cm2 of UVB was delivered. The animals were sacrificed and dorsal skin samples were harvested twenty-four hours after acute UV irradiation. For chronic treatment, animals were irradiated on their backs with a dose of 70mJ/cm2 of UVB as monitored using a radiometer, three times a week during a maximum period of 30 weeks. Dorsal skin free of any lesion was harvested after 12 weeks of UV exposure. Skin tumours larger than 5mm of diameter were harvested after a maximum period of 30 weeks and diagnosed as papillomas or squamous cell carcinomas (SCC) by an expert pathologist. Non-irradiated aged-matched mice were used as controls. Samples were directly frozen in liquid nitrogen for RNA preparation or prepared for histological and in situ hybridization analysis. For GSK0660 treatment, 200 µl of GSK0660 (625 µg/µl in 70% ethanol; Sigma, G5797) was applied topically on the back 1h prior to UV exposure.

All experiments involving animals described herein were approved by the Veterinary Office of the Canton Vaud (Switzerland) in accordance with the Federal Swiss Veterinary Office Guidelines and conform to the European Commission Directive 86/609/EEC.

### - MiR-21-3p inhibitor in vivo sub-cutaneous injections

Mice received an injection of *in vivo* inhibitor "mmu-miR-21a-3p_inh., 5'-FAM labeled" (Exiqon Inhibitor Probe Number 199900, SEQ ID NO: 7: CCATCGACTGCTGTT) *or in vivo* inhibitor mismatch control "mmu-miR-21a-3p_3MM, 5'-FAM labeled" as a control (SEQ ID NO: 9: CCCTAGACTGCTCTT) at a concentration of 20 mg/kg. Injections were performed 6h prior and 30 minutes after acute UV irradiation under isoflurane anaesthesia. Mice were scarified 24 h after UV exposure. Sacrifice was immediately followed by the dermis/epidermis separation and sample snap-freezing in liquid nitrogen.

### - Extraction of total RNA

Total RNA was isolated using TRIzol reagent (Invitrogen), according to the manufacturer protocol, and quantified using spectrophotometer at an optical density of 260nm (Nanodrop ND1000). Quality of the RNA was assessed using a BioAnalyzer (Agilent).

### - Human skin biopsies

Cutaneous SCC samples were obtained anonymously from the Department of Dermatology, University Hospital of Lausanne, Switzerland. Normal skin was from healthy adult volunteers. Pathologists diagnosed SCC. Informed consent for research was obtained prior to routine diagnostic services.

### - Cell culture, transfection and treatments

Human immortalized keratinocytes HaCaT cells were maintained in DMEM growth medium (Gibco) supplemented with 4500 mg/l glucose, 10% fetal bovine serum, 100 units/ml of penicillin G and 100 µg/ml of streptomycin. Cells were grown in a 5% CO₂ atmosphere at 37 °C. For miR-21-3p functional analysis, cells were seeded and grown for 24 h, mimic (which amino acid sequence is identical to that of mature miR-21-3p) or control (amino acid sequence of mi-R-67-3p of SEQ ID NO: 28 (UCACAACCUCCUAGAAAGAGUAGA) (Miridian, Thermo Scientific Dharmacon) were transfected to the cells at a concentration of 50 nM using the Lipofectamine RNAi max (Invitrogen). Cells were harvested 48h after transfection for gene expression analysis and 72h after transfection for lipidome analysis.

### - mRNA Microarray Data Analysis

For hybridization, GeneChip MouseGene 1.0 ST arrays (Affymetrix Inc, Tokyo, Japan) were used (Genomic Technologies Facility-GTF, CIG) to analyze the expression a set of 28,000 gene-level probe sets (750,000 unique 25-mer oligonucleotide features). After hybridization and washing, the Affymetrix GeneChip Command Console software (AGCC) was used for array scanning. All these procedures were conducted according to the manufacturer's instructions. Affymetrix Quality Control software was employed for internal array normalization (default control probes were set as internal normalization controls). Background correction, inter-chips quantile normalization and summarization were manually processed using the "Robust Multi-Array Average" ("RMA") R package. Data were ranked using the "Linear Models for Microarray Data" R package ("LIMMA") for comparison of the different experimental conditions. In "LIMMA", p-values were obtained from moderated t-statistics using Empirical Bayesian methods. P-values were then adjusted for multiple testing with Benjamini and Hochberg's method to control the false discovery rate. For mRNA expression arrays, probe sets showing a the false discovery rate < 0.05. Four technical replicates for each group (non-irradiated mice, 12 weeks and 30 weeks irradiated mice) were averaged for fold change study. Statistical significance was set to p-value < 0.001 by applying t-test analysis adjusted for multiple comparisons. Gene intensity changes higher than 1.5 were considered as biologically significant.

### - miRNA Microarray Data Analysis

Each RNA skin sample was prepared according to the Agilent's miRNA Microarray System protocol and loaded on the mouse microarray (Mouse miRNA Microarray Release 16.0) able to detect 567 mouse miRNAs (Sanger miRBase, release 10.1). Normalization procedures were based on the invariant procedure and the quantile normalization using the "normalize.quantiles" function from R package "affy" from the Bioconductor project (http://www.bioconductor.org) as described in (Pradervand S 2009). The selected invariant probes had high mean expression and low standard deviation to reduce stochastic effects. Quantile normalization assumed that the global distribution of signal intensity did not change across arrays (n=4). After normalization, the «LIMMA» package was used to define a robust linear regression for inter-conditions fold change calculation. In "LIMMA", p-values were obtained from moderated t-statistics using Empirical Bayesian methods. P-values were then adjusted for multiple testing with Benjamini and Hochberg's method to control the false discovery rate. Features showing a false discovery rate < 0.1 were considered significant for miRNA arrays. Statistical significance was set to p-value < 0.001 by applying t-test analysis adjusted for multiple comparisons. miRNA showing fold changes higher than 1.5 were selected.

Differences in major biological functions were identified using Ingenuity's pathway analysis (IPA) v2.0 (Ingenuity Systems, Redwood City, CA). The data files containing the probe identifier (gene accession numbers) and the corresponding changes in expression values (fold change (FC) and p-value) were uploaded into IPA. Pathways were considered significantly different when the right-tailed Fisher's exact test p-value threshold where below 0.005.

### - Reverse transcription and Real-Time PCR

mRNA: one µg of total RNA was reverse transcribed with random hexamere primers (Promega) using the SuperScriptII Reverse Transcriptase (Invitrogen). Real-Time PCR was performed with SYBR Green PCR Master Mix (Applied Biosystems) using Stratagene Mx3000p thermo cycler. Primer sequences are listed below. Expression was related to the expression of the house keeping genes Gapdh and Ef1a1 for mice samples and to Rp127 and Hprt for human samples and cells (GeNorm, M value <0.5). microRNA: Reverse-transcription was performed using the Exiqon miRCURY LNA™ Universal RT microRNA kit. Quantitative PCR was performed according to manufacturer protocol with microRNA LNA™ PCR primer sets and SYBR Green PCR Master Mix (Applied Biosystems). qPCR was performed on a MX3000P machine (Stratagene). miR-103 and sno234, for which the expression is stable in our model, were used as control for normalization.

Quantification of relative expression was based on the determination of the threshold cycle (Ct). Comparison of the relative expression is based on the two by two-sided Student's T-test for paired samples. Significance threshold was set at p-value < 0.05.

### - mRNA primer sequences

| Name | Forward primer | Reverse primer |
|---|---|---|
| | SEQ ID NO: 10: | SEQ ID NO: 11: |
| mmu-Gapdh | GTATGACTCCACTACGGCAAA | TTCCCATTCTCGGCTTG |
| | SEQ ID NO: 12: | SEQ ID NO: 13: |
| mmu-Ef1a1 | CCTGGCAAGCCCATGTGT | TCATGTCACGAACAGCAAAGC |
| | SEQ ID NO: 14: | SEQ ID NO: 15: |
| mmu-Pparβ/δ | CGGCAGCCTCAACATGG | AGATCCGATCGCACTTCTCATAC |
| | SEQ ID NO: 16: | SEQ ID NO: 17: |
| hsa-Pparβ/δ | GCATGAAGCTGGAGTACGAGAAG | GCATCCGACCAAAACGGATA |
| | SEQ ID NO: 18: | SEQ ID NO: 19: |
| mmu-Pparβ/δ | CGGCAGCCTCAACATGG | AGATCCGATCGCACTTCTCATAC |
| | SEQ ID NO: 20: | SEQ ID NO: 21: |
| hsa-P21 | CTGTCACTGTCTTGTACCCT | GGTAGAAATCTGTCATGCTGG |
| | SEQ ID NO: 22: | SEQ ID NO: 23: |
| hsa-P15 | CGTGGGAAAGAAGGGAAGAGT | CCCCAGACGCGCAGC |

Hsa- and mmu- Angpt14, Tgfβ-1, hsa-IL6, hsa-COX2 (has-PTGS2), hsa-Mmp1, hsa-Rp127, hsa-Hprt have been purchased from Qiagen (QuantiTect primers).

### - miRNAs primer sequences

mmu/hsa-miR-21-3p; mmu/hsa-miR-21; mmu/hsa-miR-103 and a customized hsa- and mmu-pre-miR-21 primers have been purchased from Exiqon (microRNA LNA PCR primer sets).

hsa-pri-miR-21 forward primer: SEQ ID NO: 24: TTTTGTTTTGCTTGGGAGGA hsa-pri-miR-21 reverse primer: SEQ ID NO: 25: AGCAGACAGTCAGGCAGGAT

### - miRNA fluorescent in situ hybridization with Locked nucleic acid (LNA) probes

Skin samples were fixed and cryopreserved at 4°C in 4% in paraformaldehyde and 30% sucrose overnight. Samples were then frozen in optimum cutting temperature matrix (OCT) at -80°C, further cut in 10µm sections (Cryostat, Leica), and mounted on slides. During the acetylation step, sections were washed in 0.1M of Triethanolamine/10min, then Triethanolamine/0.25% acetic anhydride/10min. 100 µl of Digoxigenin-labelled LNA probes (Exiqon) (25 nM in hybridization buffer) were added on each section and hybridization was performed O/N (16h) at 54°C (20°C below probes melting temperature) in a humid chamber. Following hybridization, sections were washed 3 times with 0.2x SCC /20min/60°C and equilibrated in TN buffer (100 mM Tris-HCl pH 7.5, 150 mM NaCl) for 5 minutes at room temperature. Sections were then blocked in TNB buffer (0.5% Blocking Reagent-Perkin Elmer in TN buffer) for 30 minutes at room temperature. To quench peroxidase activity, the slides were incubated in 3% H2O2 for 1hr and washed with TNT (0.05% Tween20 in TN buffer) 3 x 5 min. Diluted anti-DIG-POD 1:500 in TNB was applied and incubated for 30 min at room temperature. After 3 washes with TNT - 3 x 5 min slides were incubated 10 min at room temperature with diluted Cy3-Tyramide 1:50 in Amplification Reagent (TSATM Plus Cy5 Fluorescence System, Perkin Elmer). Finally slides were washed with TNT - 3 x 5 min and mounted in Mowiol.

LNA probes were designed by Exiqon:
miR-21-3p: /5DigN/GACAGCCCATCGACTGCACTGCTGTTG/3Dig_N/ (SEQ ID NO: 26 with Dig labelling at 5' and 3')
Scramble-miR: /5DigN/GTGTAACACGTCTATACGCCA/3Dig_N/ (SEQ ID NO: 27 with Dig labelling at 5' and 3')

### - Statistics summary

Unless indicated otherwise, all data are presented as the means standard errors of the mean, and statistical differences were evaluated by two-tailed Student's t-tests. For all analyses, we considered pvalue<0.01 to be statistically significant.

### Example 1. PPARβ/δ regulates the expression of UV-induced epidermal miR-21-3p, a miRNA also up regulated in human skin carcinomas

MiR-21-3p is the passenger miRNA of miR-21-5p (commonly named miR-21), a well characterized "oncomiR". Given the known induction of miR-21-5p by UV irradiation and its oncogenic role in skin squamous cell carcinomas, we tested the hypothesis that in the skin, miR-21-3p is not degraded (as passenger miRNAs are commonly thought to be) by quantifying the miR-21-3p/miR-21-5p ratio using RNA sequencing counts in various murine organs, including unchallenged skin. This ratio was strikingly higher in the skin compared to kidney, testis, brain and heart (Figure 1A). Although miR-21-3p expression remained modest compared to miR-21-5p, these data show that miR-21-3p was enriched over miR-21-5p in the skin specifically, pointing to a selective importance for miR-21-3p in the physiology of this organ. Consistent with this hypothesis, we found that miR-21-3p expression was not only up regulated in response to UV in the skin of PPARβ/δ +/+ mice (Figure 1B), but was also considerably increased in human squamous cell carcinoma biopsies compared to healthy skin (Figure 1C).

Further RT-PCR quantification highlighted that the level of miR-21-3p was induced in the chronically UV irradiated skin of PPARβ/δ +/+ mice, while UV exposure failed to up regulate miR-21-3p level in the skin of PPARβ/δ -/- mice (Figure 1B). Furthermore, the magnitude of UV-dependent increase of miR-21-3p level was reduced by pharmacological inhibition of PPARβ/δ with its antagonist GSK0660 in PPARβ/δ +/+ skin, showing that PPARβ/δ activity is required for full activation of cutaneous miR-21-3p upon UV exposure (data not shown).

Both the epidermis and the dermis are affected by UV exposure. The epidermis is directly exposed to UVA and B, while the dermis is reached by UVA only and indirectly exposed to UVB-induced changes via the epidermal-dermal cross talk.

In order to distinguish in which skin compartment was the level of miR-21-3p induced by UV exposure, in situ hybridization was performed in PPARβ/δ +/+ and -/- skin samples, and miR-21-3p levels were quantified in isolated epidermis and dermis, which successful separation was monitored using specific markers. The subcellular localization of miR-21-3p in non-irradiated skin revealed a clear preferential expression of this miRNA in the epidermis and hair follicles of PPARβ/δ +/+ skin, with low or no expression in the dermis (Figure ID). Following acute UV exposure, the level of miR-21-3p was increased in PPARβ/δ +/+ but not in the PPARβ/δ -/- epidermis, as shown by *in situ* hybridization (Figure ID), RT-qPCR (Figure IE). UV-induced miR-21-3p increase was specific to the epidermis, since its level in the dermis remained unchanged after acute UV irradiation (Figure ID). The specific localization of miR-21-3p in the epidermis could be advantageous for therapeutic applications. Of note, the expression pattern of miR-21-3p correlated with the subcellular localization and the changes observed in PPARβ/δ expression levels, which was similarly up regulated in the epidermis by acute UV exposure, while remaining stable in the dermis. In line with *these in vivo* data pointing to a specific expression of miR-21-3p in keratinocytes, miR-21-3p level was increased following activation of PPARβ/δ with its agonists GW501516 and GW0742 in the human keratinocyte cell line HaCat (Figure 1F), like the two well-characterized PPARβ/δ target genes Angpt14 and Tgfβ-1 (data not shown). Collectively, these findings revealed that miR-21-3p expression is under the control of PPARβ/δ, in murine epidermis exposed to chronic or acute UV, as well as in HaCat human keratinocytes. Furthermore, miR-21-3p likely belongs to a skin cancer molecular signature, since its expression is dramatically increased in murine and human squamous cell carcinomas.

### Example 2. MiR-21-3p mimic treated keratinocytes show a psoriasis-like molecular signature

Genome-wide microarray analysis of miR-21-3p mimic treated human HaCaT keratinocytes compared to a three-bases mistmaching miR-21-3p control sequence indicates that among the mRNA that exhibit a mimic-dependent regulation, 6.7% of them are known psoriasis-related mRNAs. The majority of them are up-regulated upon miR-21-3p mimic transfection and are also known to be up-regulated in psoriasis (Table 1).

These up-regulated mRNA include the mRNA of the cytokine-activated S100A8 protein known to be up-regulated in hyperproliferative and psoriatic epidermis *(*Nukui et al, 2008, J Cell Biochem, 104(2):453-64*)*; Bracke et al, 2013, Arch Dermatol Res, 305(6):501-512*);* as well as STAT1 mRNA shown to be up-regulated in psoriasis lesion (Hald et al, 2013, Br J Dermatol. 168(2): 302-10 *; Bracke et al, 2013 (supra));* or the chemokine mRNA CCL5 known to induce skin-infiltrating T-cells leading to inflammation in psoriasis and other inflammatory skin diseases (Canavese et al, 2010, Dermatol Sci. 58(3):171-6 *; Bracke et al, 2013 (sura))* or other mRNAs of inflammatory mediators like IL1RN, CXCL10 and CXCL 11 known to be up-regulated in psoriasis and in atopic dermatitis (Giustizieri et al, 2001, J Allergy Clin Immunol.107(5): 871-7; Shepherd, 2004, J Invest Dermatol, 122(3): 665-9 *;* Flier et al, 2001, J Pathol. 194(4): 398-405*),* suggesting a pro-psoriasis effect for miR-21-3p gain of function in HaCat keratinocytes at the molecular level.

Among the down-regulated mRNA in miR-21-3p mimic treated keratinocytes, Casp14 mRNA, which expression is known to be inhibited in the parakeratotic regions of psoriatic skin (Lippens et al, 2000, Cell Death Differ. 7(12):1218-24 *;* Bracke et al, 2013, Arch Dermatol Res, 305(6):501-512)*,* suggesting a pro-psoriasis role for miR-21-3p gain of function in HaCat keratinocytes.

**Table 1**

| **Fold change mimic / control** | **P value** | **Gene name** | **Gene description** | **Psoriasis involvement** | **Reference** |
|---|---|---|---|---|---|
| -1.598 | 2.97E-04 | **CASP14** | Caspase 14, apoptosis-related cysteine peptidase | down regulated in psoriasis | *Bracke et al, 2013 (supra); Lippens et al, 2000 (supra)* |
| 1.733 | 4.55E-02 | **UGT2B7** | UDP glucuronosyltransferase 2 family, polypeptide B7 | cornification markers | Jabbari et al, 2012, J Invest Dermatol., 132(1): 246-9 |
| 1.752 | 4.21E-03 | **KRT1** | Keratin 1 | up regulated in psoriasis | Roth et al, 2012, J Cell Sci, 125:5269-79 |
| 1.863 | 1.23E-05 | **EPGN** | Epithelial mitogen homolog (mouse) | up regulated in psoriasis | *Jabbari et al, 2012, supra* |
| 1.968 | 8.71E-04 | **S100A8** | S 100 calcium binding protein A8 | up regulated in psoriasis | *Bracke et al, 2013 (supra) ; Nukui et al, 2008 (supra* |
| 1.984 | 5.04E-07 | **STAT1** | Signal transducer and activator of transcription | up regulated in psoriasis | *Bracke et al, 2013 (supra)* |
| 2.093 | 4.29E-07 | **CCL5** | Chemokine 5 | up regulated in psoriasis | *Bracke et al, 2013 (supra); Canavese et al, 2010 (supra)* |
| 2.138 | 2.60E-07 | **CD274** | CD274 molecule | up regulated in psoriasis | *Jabbari et al, 2012, supra* |
| 2.320 | 3.74E-08 | **IL1RN** | Interleukin 1 receptor antagonist | up regulated in psoriasis | *Giustizieri et al, 2001 (supra) ; Shepherd et al, 2004 (supra)* |
| 2.465 | 5.46E-09 | **TNFSF10** | Tumor necrosis factor (ligand) superfamily, member 10 | up regulated in psoriasis | Peternel et al, 2011, Arch Dermatol Res, 303(6): 389-97 |
| 4.257 | 4.66E-06 | **CXCL11** | Chemokine ligand 11 | up regulated in psoriasis | Flier et al, 2001, J Pathol. 194(4): 398-405 |
| 7.165 | 3.44E-06 | **CXCL10** | Chemokine ligand 10 | up regulated in psoriasis | *Giustizieri et al, 2001 (supra) ; Bracke et al, 2013 (supra)* |

### Example 3. MiR-21-3p mimic treated keratinocytes show a similar expression profile of some markers mentioned in Table 1 as in various skin disorders

Regarding the involvement of some of the markers indicated in Table 1, the expression of which is modulated by miR-21-3p, in various skin disorders, it can be mentioned that:
- Casp14 deficient newborn mice shows barrier disruption, delay in cornification and high UV-sensitivity and are more prone to the development of parakeratosis *(*Hoste et al, 2013, J Invest Dermatol, 133(3):742-50*).* This also suggests a role for CASP14 in the maintenance of the skin barrier integrity that is strongly in cause in eczema, acne and other inflammatory skin diseases including as well psoriasis and in UV photodamage protection.
- Keratin 1 (KRT1), a major constituent of the intermediate filament cytoskeleton in suprabasal epidermis, is mutated in epidermolytic ichthyosis in humans. Indeed, as described in Roth et al (2012, J Cell Sci, 125(Pt 22):5269-79*),* the "absence of Krt1 causes a prenatal increase in interleukin-18 and the S100A8 and S100A9 proteins, accompanied by a barrier defect and perinatal lethality". It is also shown that transcriptome profiling revealed a Krt1-mediated gene expression signature similar to atopic eczema and psoriasis, suggesting a functional link between KRT1 and human inflammatory skin diseases.
- The S100A8 protein has also been shown to be involved in skin tumor formation *(*McNeill et al., 2014, Int J Cancer, Jan 16 *;* Hummerich et al, 2006, Oncogene, 25(1):111-21*).*
- COX2 (PTGS2) has also been linked to a large range of inflammatory skin diseases including Vitiligo (Li et al, 2009, J Dermatol Sci, 53(3):176-81), leprosy *(*Pesce et al, 2006, Am J Trop Med Hyg, 74(6):1076-7*).*
- MMP1 has been associated with wound healing of the skin *(*Stevens et al, 2012, Mol Biol Cell, 23(6):1068-79*)*

Together, these data point to a stimulatory effect of miR-21-3p on a large panel of inflammation-related markers involved in multiple skin disorders including skin cancers, psoriasis, leprosy, vitiligo, eczema, keratosis, epidermolytic ichthyosis, UV photodamages, as well as in skin inflammatory reactions like acne, allergy, UV erythema, skin ageing.

### Example 4. Inflammatory markers are up-regulated in miR-21-3p mimic treated HaCat keratinocytes

COX2 (prostaglandin-endoperoxide synthase (PTGS)) is an enzyme responsible for the formation of biological mediators called prostanoids (prostaglandins, prostacyclin and thromboxane). The mRNA of COX2 has been shown to be up-regulated in psoriatic skin *(*Yalçin et al, 2007, Anal Quant Cytol Histol, 29(6): 358-64*).*

IL-6 production has been associated with psoriatic skin (Grossman et al, 1989, Proc Natl Sci USA, 86(16):6367-71*).*

Real-time qPCR quantification of Cox2 and IL-6 mRNA shows up-regulated expression of these two inflammatory markers in miR-21-3p treated HaCat cells compared to control treated cells (Figure 2).

Thus, these observations indicate a possible pro-inflammatory role for miR-21-3p in keratinocytes.

### Example 5. Phosphatidylcholine content is up-regulated in miR-21-3p mimic treated HaCat keratinocytes

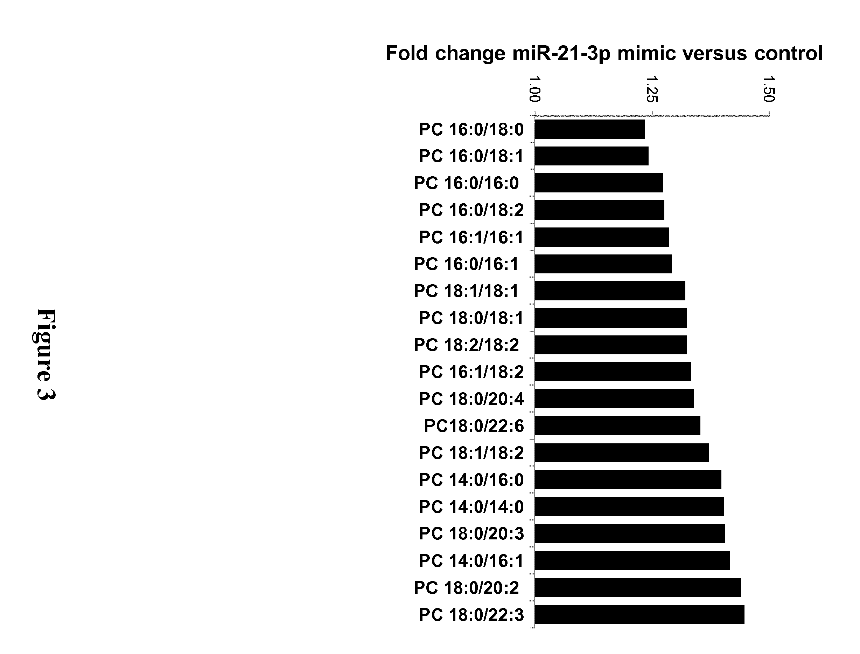

Quantification of phosphatidylcholine content in miR-21-3p mimic treated HaCat cells compared to control treated HaCat cells indicates an overall increased level of phosphatidylcholine upon miR-21-3p gain of function (Figure 3).

Previous studies showed an increase in phosphatidylcholine content in psoriatic papules compared to normal skin (Sergeev et al, 1993, Biull Eksp Biol Med., 116(9): 2 71-2*).* This observation suggests that miR-21-3p gain of function induces an increase in phosphatidylcholine in HaCat cells similar to what is observed in psoriatic skin.

### Example 6. The cell cycle inhibitors p15 and p21 are down regulated in miR-21-3p mimic treated HaCat keratinocytes

Cdkn2B (p15) and Cdkn1A (p21) are two cyclin-dependent kinase inhibitors responsible for the TGFβ-1-induced cell cycle arrest.

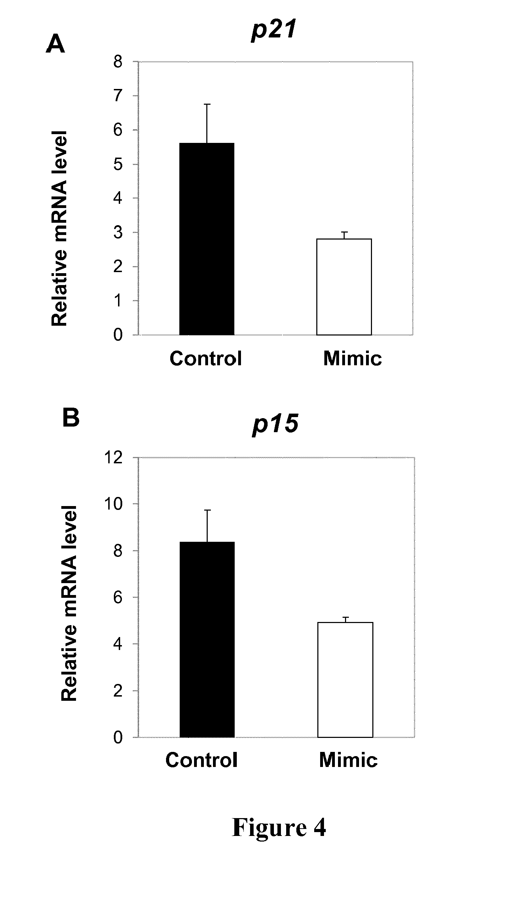

Real-time qPCR quantification of the mRNA of these cell cycle inhibitors p15 and p21 shows that they are down regulated in miR-21-3p mimic treated HaCat keratinocytes compared to control (Figure 4).

These observations suggest that miR-21-3p gain of function may stimulate cell cycle. Inhibition of cell cycle inhibitors may lead to skin hyperproliferation observed in psoriasis phenotype.

### Example 7. MMP1 is down regulated in miR-21-3p mimic treated HaCat keratinocytes (white) compared to control (black)

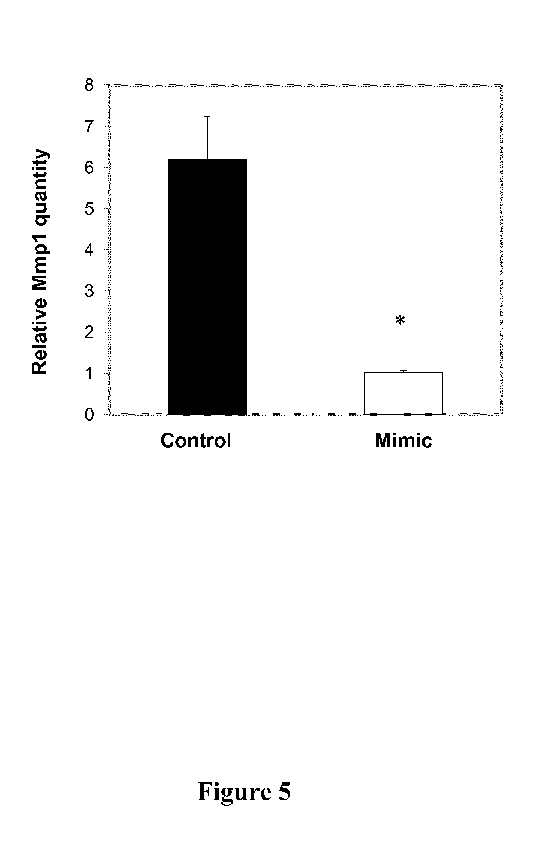

Real-time qPCR quantification of MMP1 mRNA shows that it is down regulated in miR-21-3p mimic treated HaCat cells compared to control (Figure 5).

MMP1 expression has been show as down regulated in lesional scales of patients with severe psoriasis (Flisiak et al, 2006, Acta Derm Venereol, 86(1): 17-21*)*

### Example 8. MiR-21-3p inhibitor fluorescence increases in the skin upon UV-induced inflammatory condition.

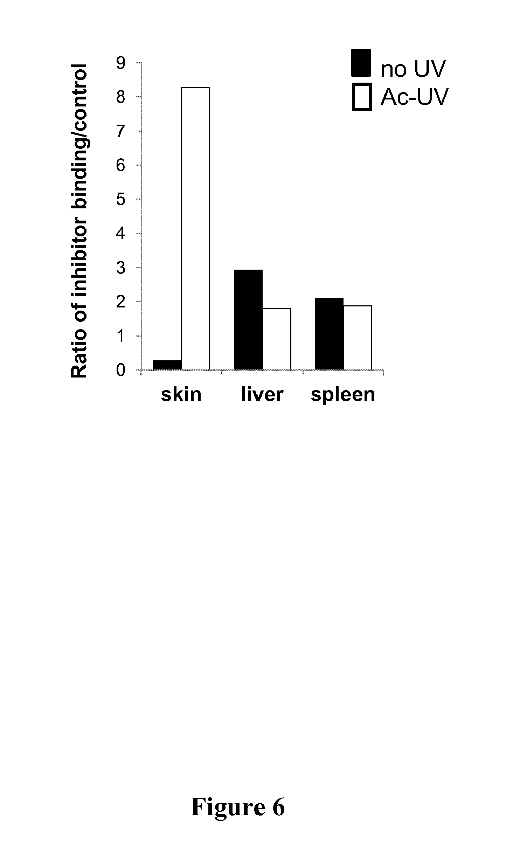

Efficient delivery of a FAM-labelled miR-21-3p inhibitor, aimed at counteracting the endogenous miR-21-3p, was monitored using the FAM fluorescence, supposed to be stabilized upon binding of the inhibitor to its target. Quantification of the fluorescence in skin, liver, spleen and muscle lysate showed that the fluorescence was decreased in liver, spleen and muscle, whereas was specifically increased in the skin in acutely irradiated mice, suggesting that miR-21-3p inhibitor was stabilized in this organ after acute UV irradiation (Figure 6).

As shown in Figure 6, miR-21-3p inhibitor fluorescence increases in the skin upon UV-induced inflammatory condition. This also addresses the skin-specificity of the miR-21-3p inhibitor delivery which accumulates in the inflamed skin rather than in spleen or liver, and would thus make miR-21-3p inhibitors advantageous as therapeutics to avoid or reduce a systemic effect and unwanted side-effects.

### Sequence listing

Human miR-21-3p (miRBase accession number: MIMAT0004494)
   SEQ ID NO: 1: CAACACCAGUCGAUGGGCUGU
Murine miR-21-3p (miRBase Accession number MIMAT0004628).
   SEQ ID NO: 2: CAACAGCAGUCGAUGGGCUGUC
Inhibitor, complementary sequence of human miR-21-3p (21 nucleotides in total)
   SEQ ID NO: 3: ACAGCCCATCGACTGGTGTTG
Human miR-21-3p inhibitor 1 (15 nucleotides in total)
   SEQ ID NO: 4: CCATCGACTGGTGTT
Human miR-21-3p inhibitor 2 (18 nucleotides in total)
   SEQ ID NO: 5: AGCCCATCGACTGGTGTT
Inhibitor, complementary sequence of murine miR-21-3p (21 nucleotides in total)
   SEQ ID NO: 6: ACAGCCCATCGACTGCTGTTG
Murine miR-21-3p inhibitor 1 (15 nucleotides in total)
   SEQ ID NO: 7: CCATCGACTGCTGTT
Murine miR-21-3p inhibitor 2 (18 nucleotides in total)
   SEQ ID NO: 8: AGCCCATCGACTGCTGTT
*in vivo* inhibitor mismatch control "mmu-miR-21a-3p_3MM, 5'-FAM labeled"
   SEQ ID NO: 9: CCCTAGACTGCTCTT, with FAM labelling in 5'
Primers for mRNA reverse transcription and quantitative PCR:
   mmu-Gapdh mRNA forward primer: SEQ ID NO: 10:
      GTATGACTCCACTACGGCAAA
   mmu-Gapdh mRNA reverse primer SEQ ID NO: 11: TTCCCATTCTCGGCTTG
   mmu-Efla1 mRNA forward primer: SEQ ID NO: 12: CCTGGCAAGCCCATGTGT
   mmu-Efla1 mRNA reverse primer: SEQ ID NO: 13:
      TCATGTCACGAACAGCAAAGC
   mmu-Pparβ/δ mRNA forward primer: SEQ ID NO: 14: CGGCAGCCTCAACATGG
   mmu-Pparβ/δ mRNA reverse primer: SEQ ID NO: 15:
      AGATCCGATCGCACTTCTCATAC
   hsa-Pparβ/δ mRNA forward primer: SEQ ID NO: 16:
      GCATGAAGCTGGAGTACGAGAAG
   hsa-Pparβ/δ mRNA reverse primer: SEQ ID NO: 17: GCATCCGACCAAAACGGATA
   mmu-Pparβ/δ mRNA forward primer: SEQ ID NO: 18: CGGCAGCCTCAACATGG
   mmu-Pparβ/δ mRNA reverse primer: SEQ ID NO: 19:
      AGATCCGATCGCACTTCTCATAC
   hsa-P21 mRNA forward primer: SEQ ID NO: 20: CTGTCACTGTCTTGTACCCT
   hsa-P21 mRNA reverse primer: SEQ ID NO: 21: GGTAGAAATCTGTCATGCTGG
   hsa-P15 mRNA forward primer: SEQ ID NO: 22: CGTGGGAAAGAAGGGAAGAGT
   hsa-P15 mRNA reverse primer: SEQ ID NO: 23: CCCCAGACGCGCAGC
   hsa-pri-miR-21 forward primer: SEQ ID NO: 24: TTTTGTTTTGCTTGGGAGGA
   hsa-pri-miR-21 reverse primer : SEQ ID NO: 25: AGCAGACAGTCAGGCAGGAT
miR-21-3p LNA probe: SEQ ID NO: 26: GACAGCCCATCGACTGCACTGCTGTTG, with Dig labelling in 5' and 3'
   Scramble-miR LNA probe: SEQ ID NO: 27: GTGTAACACGTCTATACGCCA, with Dig labelling in 5' and 3'
mi-R-67-3p:
   SEQ ID NO: 28: UCACAACCUCCUAGAAAGAGUAGA

## Claims

1. A miR-21-3p inhibitor for use in the prevention and/or treatment of skin disorders.

2. The miR-21-3p inhibitor for use according to claim 1, wherein said inhibitor is an oligonucleotide hybridizing to:
(i) the nucleic acid sequence of miR-21-3p or a fragment thereof, in particular human miR-21-3p of SEQ ID NO: 1 or murine miR-21-3p of SEQ ID NO: 2 or a variant thereof or a fragment thereof, and/or
(ii) the seed region of miR-21-3p, in particular a sequence corresponding to nucleotides 2 to 7 of SEQ ID NO: 1 or nucleotides 2 to 7 of SEQ ID NO: 2 or a variant thereof, and/or
(iii) a fragment of at least 10 contiguous nucleotides from the nucleic acid sequence of miR-21-3p, in particular human miR-21-3p of SEQ ID NO: 1 or murine miR-21-3p of SEQ ID NO: 2 or a variant thereof, optionally comprising the seed region of miR-21-3p, in particular a sequence corresponding to nucleotides 2 to 7 of SEQ ID NO: 1 or nucleotides 2 to 7 of SEQ ID NO: 2 or a variant thereof.

3. The miR-21-3p inhibitor for use according to any one of claims 1 to 2, wherein said inhibitor is an oligonucleotide complementary to:
(i) the nucleic acid sequence of miR-21-3p or a fragment thereof, in particular human miR-21-3p of SEQ ID NO: 1 or murine miR-21-3p of SEQ ID NO: 2 or a variant thereof or a fragment thereof, and/or
(ii) the seed region of miR-21-3p, in particular a sequence corresponding to nucleotides 2 to 7 of SEQ ID NO: 1 or nucleotides 2 to 7 of SEQ ID NO: 2 or a variant thereof, and/or
(iii) a fragment of at least 10 contiguous nucleotides from the nucleic acid sequence of miR-21-3p, in particular human miR-21-3p of SEQ ID NO: 1 or murine miR-21-3p of SEQ ID NO: 2 or a variant thereof, optionally comprising the seed region of miR-21-3p, in particular a sequence corresponding to nucleotides 2 to 7 of SEQ ID NO: 1 or nucleotides 2 to 7 of SEQ ID NO: 2 or a variant thereof.

4. The miR-21-3p inhibitor for use according to any one of claims 1 to 3, wherein said inhibitor is an oligonucleotide comprising any one of:
(i) SEQ ID NO: 3, or a variant thereof, or a fragment thereof optionally comprising nucleotides 15 to 20 of SEQ ID NO: 3,
(ii) a fragment of at least 10 contiguous nucleotides from SEQ ID NO: 3 optionally comprising nucleotides 15 to 20 of SEQ ID NO: 3,
(iii) SEQ ID NO: 4 or SEQ ID NO: 5,
(iv) SEQ ID NO: 6, or a variant thereof, or a fragment thereof optionally comprising nucleotides 15 to 20 of SEQ ID NO: 6,
(v) a fragment of at least 10 contiguous nucleotides from SEQ ID NO: 6 optionally comprising nucleotides 15 to 20 of SEQ ID NO: 6,
(vi) SEQ ID NO: 7 or SEQ ID NO: 8.

5. The miR-21-3p inhibitor for use according to any one of claims 1 to 4, wherein said inhibitor is a modified oligonucleotide such as a LNA-modified oligonucleotide and/or wherein said oligonucleotide is chemically linked to cholesterol.

6. The miR-21-3p inhibitor for use according to any one of claims 1 to 5, wherein said skin disorder is an inflammatory skin disorder.

7. The miR-21-3p inhibitor for use according to any one of claims 1 to 6, wherein said inflammatory skin disorder is selected from psoriasis, dermatitis, acne, rosacea, Stevens-Johnson syndrome, toxic epidermal necrolysis, systemic lupus erythematosus, skin allergies, atopic eczema, parakeratosis.

8. The miR-21-3p inhibitor for use according to any one of claims 1 to 7, wherein said inflammatory skin disorder is psoriasis or dermatitis.

9. The miR-21-3p inhibitor for use according to any one of claims 1 to 8, wherein said skin disorder is keratosis or a skin cancer.

10. The miR-21-3p inhibitor for use according to any one of claims 1 to 9, wherein said skin disorder is selected from leprosy, vitiligo, epidermolytic ichthyosis, UV photodamages, topical allergy, UV erythema, skin ageing, and wounds.

11. An *ex-vivo* method of prognosis and/or diagnosis of a skin disorder in a subject comprising:
a) Providing a biological sample from a subject;
b) Determining the level of miR-21-3p, in said sample;
c) Comparing the level of miR-21-3p determined in step b) with the level of miR-21-3p in a control sample:
wherein an increased level of miR-21-3p determined in step b) compared to the control sample is indicative of the subj ect being at risk for developing, or having, a skin disorder.

12. A miR-21-3p inhibitor comprising an oligonucleotide comprising any one of:
(i) SEQ ID NO: 3, or a variant thereof, or a fragment thereof optionally comprising nucleotides 15 to 20 of SEQ ID NO: 3,
(ii) a fragment of SEQ ID NO: 3 optionally comprising nucleotides 15 to 20 of SEQ ID NO: 3,
(iii) SEQ ID NO: 4 or SEQ ID NO: 5,
(iv) SEQ ID NO: 6, or a variant thereof, or a fragment thereof optionally comprising nucleotides 15 to 20 of SEQ ID NO: 6,
(v) a fragment of SEQ ID NO: 6 optionally comprising nucleotides 15 to 20 of SEQ ID NO: 6,
(vi) SEQ ID NO: 7 or SEQ ID NO: 8.

13. A miR-21-3p inhibitor according to claim 12, wherein said oligonucleotide is a LNA-modified oligonucleotide, wherein the ribose ring of one or more nucleotides of said LNA-modified oligonucleotide is locked by a methylene bridge connecting the 2'-O atom and the 4'-C atom and/or wherein said oligonucleotide is chemically linked to cholesterol.

14. A miR-21-3p inhibitor according to any one of claims 12 to 13 for use as a medicament.

15. A pharmaceutical composition comprising a miR-21-3p inhibitor according to any one of claims 12 to 14 or a vector comprising a nucleic acid encoding said miR-21-3p inhibitor, and a pharmaceutically acceptable carrier.
